# EUROPEAN PATENT SPECIFICATION

(11) **EP 3 603 562 B1**
(45) Date of publication and mention of the grant of the patent: **29.06.2022**
(21) Application number: 18776589.6
(22) Date of filing: 15.01.2018
(51) Int. Cl.: A61B 90/25, A61B 90/20, A61B 90/50

(54) **MEDICAL OBSERVATION APPARATUS AND OBSERVATION FIELD CORRECTION METHOD**
VORRICHTUNG ZUR MEDIZINISCHEN BEOBACHTUNG UND BEOBACHTUNGSFELDKORREKTURVERFAHREN
APPAREIL D'OBSERVATION MÉDICALE ET PROCÉDÉ DE CORRECTION DE CHAMP D'OBSERVATION

(30) Priority: 28.03.2017 JP 2017062941
(43) Date of publication of application: 05.02.2020
(73) Proprietor: Sony Olympus Medical Solutions Inc., Tokyo 192-0904 (JP)
(72) Inventor: TAGUCHI, Hidenori, Hachioji-shi, Tokyo 192-0904 (JP)
(74) Representative: D Young & Co LLP
(86) International application number: PCT/JP2018/000812
(87) International publication number: WO 2018/179681

(56) References cited:
- EP-A1- 3 144 711
- WO-A1-2015/135057
- DE-A1-102014 224 044
- JP-A- H10 118 015
- JP-A- 2009 527 267
- JP-A- 2017 038 285
- US-A- 6 036 637
- US-A1- 2014 005 475

## Description

### Technical Field

The present disclosure relates to a medical observation apparatus and an observation visual field correction method.

### Background Art

An observation apparatus for enlarging and observing an affected part has been used in surgery (which is so-called microsurgery) performed on a fine region, such as neurosurgery or the like. The observation apparatus is formed in a form in which a microscope unit is supported by an arm portion (supporting unit).

As the observation apparatus, an observation apparatus (hereinafter, also referred to as an optical observation apparatus) including an optical microscope unit has been mainly used. In a surgical operation using the optical observation apparatus, an operator performs various treatments on an affected part by using a surgical tool while enlarging and observing the affected part by directly looking into the microscope unit through an eyepiece provided on the microscope unit.

Meanwhile, in recent years, in accordance with advancement in image processing technology and realization of improvement in video resolution, an observation apparatus (hereinafter, also referred to as an electronic imaging observation apparatus) including an image sensor and an electronic imaging microscope unit which can electronically capture an image of an affected part has been developed (for example, see Patent Literature 1). In a surgical operation using the electronic imaging observation apparatus, a video captured by the microscope unit is appropriately enlarged and displayed on a display device. An operator performs various treatments on the affected part by using a surgical tool while checking a state of the affected part and the surgical tool through the video (displayed video) displayed on the display device.

In the present specification, a visual field of an operator obtained by a microscope unit of an observation apparatus is referred to as an observation visual field. In a case of the optical observation apparatus, the observation visual field corresponds to a range in which the operator can perform observation by looking into the optical microscope unit. In a case of the electronic imaging observation apparatus, the observation visual field corresponds to a range (a range of a video displayed on the display device) of the displayed video.

### Citation List

### Patent Literature

Patent Literature 1: WO 2015/046081 A
Patent Literature 2: JP H09-28663 A

### Disclosure of Invention

### Technical Problem

In a surgical operation using an observation apparatus, a microscope unit is frequently operated during the surgical operation in order to observe an affected part in various directions or change an enlargement magnification to observe the affected part. In this case, in the observation apparatus, since the affected part is enlarged and observed, an observation visual field is greatly moved even by a fine operation of the microscope unit. Therefore, it is apprehended that the affected part is away from a region near the center of the observation visual field. When the affected part is far away from the region near the observation visual field, an operation of finely adjusting a position and a posture of the microscope unit so that the affected part is positioned in the region near the center of the observation visual field, is required. The surgical operation is stopped due to the operation described above, which obstructs a smooth surgical operation and places a mental burden on an operator.

Here, as a technology for supporting an endoscopic surgical operation, Patent Literature 2 discloses an endoscope apparatus including position detecting means which acquires position information of a surgical tool based on a video captured by an endoscope, and control means which drives an imaging optical system of the endoscope to change an image capturing range so that a distal end portion of the surgical tool is positioned at substantially the center of a displayed video, based on the position information. In the endoscope apparatus, the image capturing range can be automatically changed so that the distal end portion of the surgical tool is within the image capturing range of the endoscope at all times. With this arrangement, there is no need to operate the endoscope by hand to obtain a desired video, which enables a smooth surgical operation. When the technology described in Patent Literature 2 is applied to the observation apparatus, there is a possibility that the problem caused by the movement of the observation visual field of the microscope unit described above can be solved.

However, the technology described in Patent Literature 2 relates to the endoscope apparatus. Therefore, even when the technology is applied to the observation apparatus as it is, the same effect may not be obtained.

In this regard, the present disclosure suggests a new and improved medical observation apparatus and observation visual field connection method, which enable a smooth surgical operation.

US 6036637 A discloses a treating system includes an endoscope to be inserted into a body of a patient for supplying an endoscopic image of an internal part of the body of the patient, and a treating device to be inserted into the body of the patient for treating an affected area while observing the endoscopic image supplied by the endoscope. The system further includes a holder for holding the endoscope, an image pickup section including an image-pickup optical system for picking up the endoscopic image supplied by the endoscope, and a display for displaying the endoscopic image picked up by the image pickup section. A position detector is provided for detecting position data of an object observed through the endoscope, and a view field switching device is provided for switching a field of view of the endoscopic image picked up by the image pickup section. In addition, a controller is provided for monitoring whether the treating device is operating and for prohibiting the view field switching device from switching the field of view when the treating device is detected to be operating.

### Solution to Problem

Embodiments of the present invention are defined by the appended Claims.

### Advantageous Effects of Invention

According to the present disclosure described above, a smoother surgical operation can be enabled. The effects of the present invention are not limited to those described above, and along with the effects described above or instead of the effects described above, any effect described in the present specification or another effect which can be understood from the present specification may be exhibited.

### Brief Description of Drawings

FIG. 1 is a view schematically illustrating an example of a configuration of an observation system and an observation apparatus according to a first embodiment.
FIG. 2 is a view illustrating a state in which a surgical operation using an observation system illustrated in FIG. 1 is performed.
FIG. 3 is a view schematically illustrating an example of a positional relation between the brain and a microscope unit in a neurosurgery.
FIG. 4 is a block diagram illustrating an example of a functional configuration of a control device according to the first embodiment.
FIG. 5 is a view for describing an operation at the time of changing an observation visual field in a general existing observation apparatus which does not have an observation visual field correction function according to the first embodiment.
FIG. 6 is a view for describing the operation at the time of changing the observation visual field in the general existing observation apparatus which does not have the observation visual field correction function according to the first embodiment.
FIG. 7 is a view for describing an operation at the time of changing an observation visual field in the observation apparatus according to the first embodiment.
FIG. 8 is a view for describing the operation at the time of changing the observation visual field in the observation apparatus according to the first embodiment.
FIG. 9 is a flowchart illustrating an example of a processing procedure of an observation visual field correction method according to the first embodiment.
FIG. 10 is a block diagram illustrating an example of a functional configuration of a control device according to a second embodiment.
FIG. 11 is a flowchart illustrating an example of a processing procedure of an observation visual field correction method according to the second embodiment.

### Best Mode for Carrying Out the Invention

Preferred embodiments of the present disclosure will be described in detail with reference to the accompanying drawings. In the present specification and the drawings, components having substantially the same function are denoted by the same reference numeral, thereby omitting an overlapping description.

The description will be provided in the following order.
1. First Embodiment
   1-1. Configuration of Observation System and Observation Apparatus
   1-2. Functional Configuration of Control Device
   1-3. Observation Visual Field Correction Method
2. Second Embodiment
   2-1. Functional Configuration of Control Device
   2-2. Observation Visual Field Correction Method
3. Modified Example
   3-1. Another Method for Performing Surgical Tool Recognizing Processing and Observation Visual Field Center Target Determining Processing
   3-2. Another Example of Configuration of Observation Apparatus
4. Appendix

In the description provided below, a user using an observation system and an observation apparatus to be described later will be referred to as an operator for convenience. However, the operator is not limited to the user using the observation system and the observation apparatus, and a subject who uses the observation system and the observation apparatus may also be an assistant, a nurse, or other medical staffs.

### (1. First Embodiment)

### (1-1. Configuration of Observation System and Observation Apparatus)

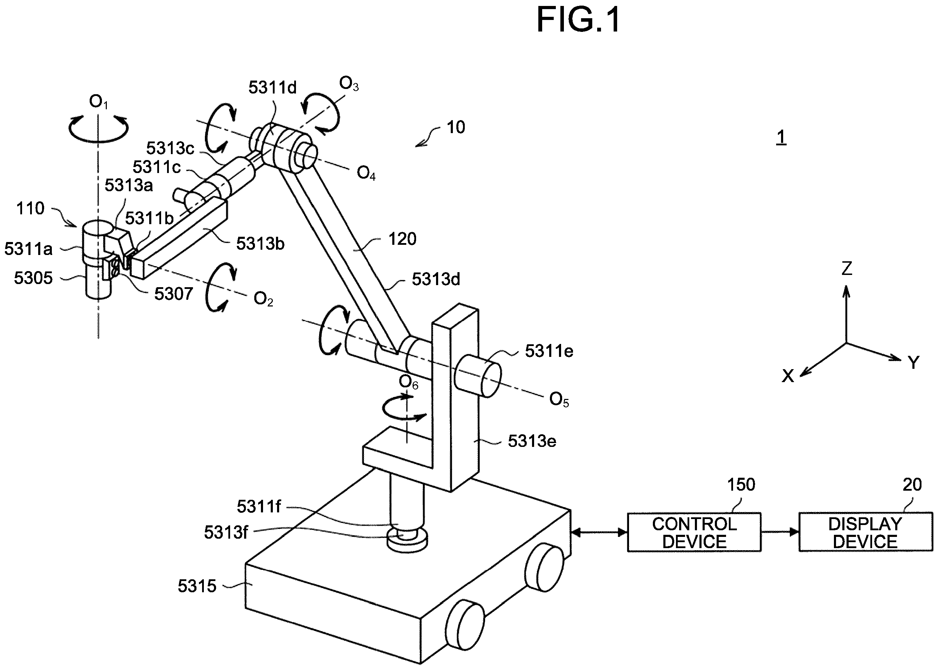

A configuration of an observation system and an observation apparatus according to a first embodiment of the present disclosure will be described with reference to FIG. 1. FIG. 1 is a view schematically illustrating an example of a configuration of an observation system and an observation apparatus according to the first embodiment.

Referring to FIG. 1, an observation system 1 according to the first embodiment is constituted by an electronic imaging observation apparatus 10 for enlarging and observing an affected part of a patient and a display device 20 which displays a video captured by the observation apparatus 10. The observation system 1 is a medical observation system for observing an observation target portion (surgical operation target portion or examination target portion) which is a part of the body of the patient at the time of performing a medical treatment such as a surgical operation, an examination, or the like. At the time of the surgical operation or the examination, the operator observes the observation target portion through the video captured by the observation apparatus 10 and displayed on the display device 20, and performs various treatments on the observation target portion as necessary. Hereinafter, a case where the surgical operation is performed by using the observation system 1 will be described by way of example, and the observation target portion (surgical operation target portion) will also be referred to as an affected part.

### (Display Device)

The display device 20 displays a video captured by the observation apparatus 10 according to a control by a control device 150 of the observation apparatus 10 to be described later. The display device 20 is installed in a place where the operator can see the display device 20 in the operating room, such as, for example, a wall of the operating room or the like. A type of the display device 20 is not particularly limited. As the display device 20, various known display devices such as, for example, a cathode ray tube (CRT) display device, a liquid crystal display device, a plasma display device, an electroluminescence (EL) display device, and the like may be used. Further, the display device 20 need not necessarily be installed in the operating room, and may be mounted in a device which is used while being attached on the body of the operator, like a head mounted display (HMD) or a spectacle-type wearable device.

As will be described later, for example, when an imaging unit of a microscope unit 110 of the observation apparatus 10 is implemented as a stereo camera, or when the imaging unit is configured to be able to perform high-resolution image capturing, a display device 20 which can display a three-dimensional image (3D) or display a high-resolution image can be used accordingly.

### (Observation Apparatus)

The observation apparatus 10 includes the microscope unit 110 for enlarging and observing the affected part of the patient, an arm portion 120 (supporting unit 120) which supports the microscope unit 110, a base portion 5315 connected to a proximal end of the supporting unit 120 to support the microscope unit 110 and the supporting unit 120, and the control device 150 which controls operations of the observation system 1 and the observation apparatus 10.

In the description provided below, a direction perpendicular to a floor surface on which the observation apparatus 10 is installed is defined as a Z-axis direction. The Z-axis direction will also be referred to as an up-down direction. Further, a direction which is orthogonal to the Z-axis direction and in which the supporting unit 120 extends from the base portion 5315 is defined as an X-axis direction. The X-axis direction will also be referred to as a front-back direction. Further, a direction orthogonal to both of the X-axis direction and the Z-axis direction is defined as a Y-axis direction. The Y-axis direction will also be referred to as a left-right direction. In addition, a plane parallel to an X-Y plane will be referred to as a horizontal plane and a direction parallel to the horizontal plane will also be referred to as a horizontal direction.

### (Base Portion)

The base portion 5315 supports the microscope unit 110 and the supporting unit 120. The base portion 5315 includes a trestle having a plate shape, and a plurality of casters provided on a lower surface of the trestle. One end of the supporting unit 120 is connected to an upper surface of the trestle, and the microscope unit 110 is connected to the other end (distal end) of the supporting unit 120 extending from the trestle. Further, the observation apparatus 10 is configured to be brought into contact with the floor surface through the casters and be movable on the floor surface by using the casters.

### (Microscope unit)

The microscope unit 110 is electronic imaging microscope unit. In the illustrated example, an optical axis direction of the microscope unit 110 is substantially the same as the Z-axis direction. The microscope unit 110 is constituted by a cylindrical portion 5305 which is a frame having a substantially cylindrical shape, and an imaging unit (not illustrated) provided in the cylindrical portion 5305.

Light (observation light) from an observation target is incident from an opening surface of a lower end of the cylindrical portion 5305 on the imaging unit. The imaging unit is constituted by an image sensor and an optical system which collects the observation light in the image sensor, and the observation light incident on the imaging unit is collected on a light receiving surface of the image sensor through the optical system. The observation light is photoelectrically converted by the image sensor, thereby acquiring data regarding a video of the observation target. Video data acquired by the imaging unit are transmitted to the control device 150.

It should be noted that the imaging unit may also include a driving mechanism which moves a zoom lens and a focus lens of the optical system along an optical axis. The zoom lens and the focus lens appropriately are moved by the driving mechanism, thereby making it possible to adjust an enlargement magnification of the captured image and a focal point at the time of image capturing, respectively. Further, the imaging unit can have various functions that the electronic imaging microscope unit can generally have, such as an auto exposure (AE) function, an auto focus (AF) function, and the like.

In addition, the imaging unit may be implemented as a so-called single-plate type imaging unit including one image sensor, or may be implemented as a so-called multi-plate type imaging unit including a plurality of image sensors. When the imaging unit is configured as the multi-plate type imaging unit, for example, the imaging unit may include a pair of image sensors for acquiring, respectively, an image signal for the right eye and an image signal for the left eye, which corresponds to a three-dimensional view (3D displaying). As the 3D displaying is performed, the operator can accurately grasp a depth of a biological tissue in the affected part. In the case where the imaging unit is configured as the multi-plate type imaging unit, a plurality of optical systems can each be provided for each image sensor. As the image sensor mounted in the imaging unit, various known image sensors such as a complementary metal oxide semiconductor (CMOS) image sensor, a charge coupled device (CCD) image sensor, or the like can be used.

An operating unit 5307 for controlling an operation of the microscope unit 110 is provided on an outer wall of the cylindrical portion 5305 of the microscope unit 110. The operating unit 5307 is implemented by, for example, a joystick, a switch, or the like.

For example, a zoom switch (zoom SW) and a focus switch (focus SW) can be provided in the operating unit 5307. The operator can input an instruction for adjusting the enlargement magnification and the focal point in the microscope unit 110, respectively, through the zoom SW and the focus SW. In the imaging unit, the driving mechanism of the imaging unit appropriately moves the zoom lens and the focus lens according to an instruction input through the zoom SW and the focus SW, thereby making it possible to adjust the enlargement magnification and the focal point. Hereinafter, an operation of changing, by the operator, the enlargement magnification (that is, the enlargement magnification in the imaging unit) in the microscope unit 110 is also referred to as a zoom operation. The zoom operation can be an operation of specifying the enlargement magnification through the zoom SW.

In addition, for example, an operation mode switch (operation mode SW) can be provided in the operating unit 5307. The operator can input an instruction for switching an operation mode of the supporting unit 120 into any one of a free mode and a fixed mode through the operation mode SW. Here, the fixed mode is an operation mode in which rotation around each rotational axis of the supporting unit 120 is restricted by a brake, such that a position and a posture of the microscope unit 110 are fixed. The free mode is an operation mode in which the brake is released, such that each rotational axis of the supporting unit 120 can freely rotate. For example, in the free mode, the position and the posture of the microscope unit 110 can be adjusted by a direct operation of the operator. Here, the direct operation means an operation in which the operator grips the microscope unit 110 by hand to directly move the microscope unit 110. For example, the operation mode of the supporting unit 120 is the free mode while the operator presses the operation mode SW in the operating unit 5307, and the operation mode of the supporting unit 120 is the fixed mode while the operator keeps his/her hands off the operation mode SW.

As described above, when the operator moves the microscope unit 110, a direct operation, that is, an aspect in which the operator moves the microscope unit 110 in a state in which the operator holds the cylindrical portion 5305 by gripping the cylindrical portion 5305 is assumed. Therefore, it is preferable that the operating unit 5307 (in particular, the operation mode SW) be provided at a position at which the operator can easily operate the operating unit 5307 by using his/her finger in a state in which the operator grips the cylindrical portion 5305 so that the operator can operate the operating unit 5307 even while the operator moves the cylindrical portion 5305.

### (Supporting unit)

The supporting unit 120 three-dimensionally moves the microscope unit 110 and fixedly supports the position and the posture of the microscope unit 110 after the movement. In the first embodiment, the supporting unit 120 is implemented as an arm having six degrees of freedom. However, a second embodiment is not limited to the example described above, and the supporting unit 120 may have a different number of degrees of freedom as long as the supporting unit 120 is configured to be able to appropriately move the microscope unit 110 according to an intended use.

In the supporting unit 120, six rotational axes (a first axis O₁, a second axis O₂, a third axis O₂, a fourth axis O₄, a fifth axis O₅, and a sixth axis O₆) corresponding to the six degrees of freedom are provided. Hereinafter, for convenience of explanation, members configuring each rotational axis will be collectively referred to as a rotational axis portion. For example, the rotational axis portion can be constituted by a bearing, a shaft rotatably inserted into the bearing, a brake which restricts the rotation around the rotational axis, and the like.

The supporting unit 120 is configured in a form in which a plurality of rings (first arm portion 5313a to sixth arm portion 5313f) are rotatably connected to one another by a plurality of rotational axis portions (first rotational axis portion 5311a to sixth rotational axis portion 5311f) corresponding to the six rotational axes.

The first rotational axis portion 5311a has a substantially cylindrical shape, and the first rotational axis portion 5311a supports an upper end of the cylindrical portion 5305 of the microscope unit 110 to be rotatable around a rotational axis (first axis O₁) parallel to a central axis of the cylindrical portion 5305, at a distal end (lower end) of the first rotational axis portion 5311a. Here, the first rotational axis portion 5311a can be configured so that the first axis O₁ coincides with an optical axis of the imaging unit of the microscope unit 110. In such a configuration, it is possible to change a visual field so as to rotate the captured image by rotating the microscope unit 110 around the first axis O₁.

The first arm portion 5313a fixedly supports the first rotational axis portion 5311a at a distal end of the first arm portion 5313a. In detail, the first arm portion 5313a is a rod-shaped member having a substantial L-letter shape, has one side extending in a direction orthogonal to the first axis O₁, and is connected to the first rotational axis portion 5311a so that an end portion of the side abuts an upper end portion of an outer circumference of the first rotational axis portion 5311a, the one side being adjacent to the distal end of the first arm portion 5313a. The second rotational axis portion 5311b is connected to an end portion of the other side adjacent to a proximal end of the first arm portion 5313a having a substantial L-letter shape.

The second rotational axis portion 5311b has a substantially cylindrical shape, and the second rotational axis portion 5311b supports the proximal end of the first arm portion 5313a to be rotatable around a rotational axis (second axis O₂) orthogonal to the first axis O₁, at a distal end of the second rotational axis portion 5311b. The distal end of the second arm portion 5313b is fixedly connected to a proximal end of the second rotational axis portion 5311b.

The second arm portion 5313b is a rod-shaped member having a substantial L-letter shape and has a side extending in a direction orthogonal to the second axis O₂, and an end portion of the side is fixedly connected to the proximal end of the second rotational axis portion 5311b, the side being adjacent to the distal end of the second arm portion 5313b. The third rotational axis portion 5311c is connected to the other side adjacent to a proximal end of the second arm portion 5313b having a substantial L-letter shape.

The third rotational axis portion 5311c has a substantially cylindrical shape, and the third rotational axis portion 5311c supports the proximal end of the second arm portion 5313b to be rotatable around a rotational axis (third axis O₃) orthogonal to the first axis O₁ and the second axis O₂, at a distal end of the third rotational axis portion 5311c. A distal end of the third arm portion 5313c is fixedly connected to a proximal end of the third rotational axis portion 5311c. It is possible to move the microscope unit 110 so as to change a position of the microscope unit 110 in the horizontal plane by rotating components including the microscope unit 110 around the second axis O₂ and the third axis O₃, the components being adjacent to the distal end. In other words, it is possible to move a visual field of the captured image in a plane by controlling rotations around the second axis O₂ and the third axis O₃.

The third arm portion 5313c is configured so that the distal end of the third arm portion 5313c has a substantially cylindrical shape, and a proximal end of the third rotational axis portion 5311c is fixedly connected to the distal end having the cylindrical shape so that the proximal end of the third rotational axis portion 5311c and the distal end of the third arm portion 5313c have substantially the same central axis. A proximal end of the third arm portion 5313c has a prism shape and the fourth rotational axis portion 5311d is connected to the distal end of the third arm portion 5313c.

The fourth rotational axis portion 5311d has a substantially cylindrical shape, and the fourth rotational axis portion 5311d supports the proximal end of the third arm portion 5313c to be rotatable around a rotational axis (fourth axis O₄) orthogonal to the third axis O₃, at a distal end of the fourth rotational axis portion 5311d. A distal end of the fourth arm portion 5313d is fixedly connected to a proximal end of the fourth rotational axis portion 5311d.

The fourth arm portion 5313d is a rod-shaped member extending substantially linearly. The fourth arm portion 5313d extends so as to be orthogonal to the fourth axis O₄ and is fixedly connected to the fourth rotational axis portion 5311d so that an end portion of the distal end of the fourth arm portion 5313d abuts a side of the fourth rotational axis portion 5311d, the side having a substantially cylindrical shape. The fifth rotational axis portion 5311e is connected to a proximal end of the fourth arm portion 5313d.

The fifth rotational axis portion 5311e has a substantially cylindrical shape, and the fifth rotational axis portion 5311e supports the proximal end of the fourth arm portion 5313d to be rotatable around a rotational axis (fifth axis O₅) parallel to the fourth axis O₄, at a distal end of the fifth rotational axis portion 5311e. A distal end of the fifth arm portion 5313e is fixedly connected to a proximal end of the fifth rotational axis portion 5311e. The fourth axis O₄ and the fifth axis O₅ are rotational axes around which the microscope unit 110 can be moved in the up-down direction. It is possible to adjust a height of the microscope unit 110, that is, a distance between the microscope unit 110 and the observation target by rotating components including the microscope unit 110 around the fourth axis O₄ and the fifth axis O₅, the components being adjacent to the distal end.

The fifth arm portion 5313e is configured by combining a first member having a substantial L-letter shape in which one side extends in a vertical direction and the other side extends in the horizontal direction, and a rod-shaped second member extending vertically downward from a portion of the first member, the portion extending in the horizontal direction. The proximal end of the fifth rotational axis portion 5311e is fixedly connected in the vicinity of an upper end of a portion of the first member of the fifth arm portion 5313e, the portion of the first member extending in the vertical direction. The sixth rotational axis portion 5311f is connected to a proximal end (lower end) of the second member of the fifth arm portion 5313e.

The sixth rotational axis portion 5311f has a substantially cylindrical shape, and the sixth rotational axis portion 5311f supports a proximal end of the fifth arm portion 5313e to be rotatable around a rotational axis (sixth axis O₆) parallel to the vertical direction, at a distal end of the sixth rotational axis portion 5311f. A distal end of the sixth arm portion 5313f is fixedly connected to a proximal end of the sixth rotational axis portion 5311f.

The sixth arm portion 5313f is a rod-shaped member extending in the vertical direction, and a proximal end of the sixth arm portion 5313f is fixedly connected to an upper surface of the base portion 5315.

A range in which the first rotational axis portion 5311a to the sixth rotational axis portion 5311f can rotate is appropriately set so that the microscope unit 110 can perform a desired motion. As a result, the supporting unit 120 having the configuration described above can implement the total six degrees of freedom including three degrees of freedom in translational motion and three degrees of freedom in rotational motion of the microscope unit 110. As described above, the supporting unit 120 is configured so that the six degrees of freedom in motion of the microscope unit 110 is implemented, such that the position and the posture of the microscope unit 110 can be freely controlled within a movable range of the supporting unit 120. Therefore, the affected part can be observed at various angles, and thus it is possible to enable a smoother surgical operation.

An actuator on which a driving mechanism such as a motor or the like, an encoder which detects a rotational angle of each joint portion, and the like are mounted can be provided in each of the first rotational axis portions 5311a to the sixth rotational axis portion 5311f of the supporting unit 120. Further, driving of an actuator provided in each of the first rotational axis portion 5311a to the sixth rotational axis portion 5311f is appropriately controlled by the control device 150, such that a posture of the supporting unit 120, that is, the position and the posture of the microscope unit 110 can be controlled. In detail, the control device 150 can identify a current posture of the supporting unit 120, and a current position and a current posture of the microscope unit 110 based on information on a rotational angle of each rotational axis portion detected by the encoder. The control device 150 calculates a control value (for example, a rotational angle, a generation torque, or the like) with respect to each rotational axis portion for implementing movement of the microscope unit 110 according to an operation input from the operator by using the identified information, and drives the driving mechanism of each rotational axis portion according to the control value. In this case, the method of controlling the supporting unit 120 by the control device 150 is not limited, and various known control methods such as a force control, a position control, and the like may be applied. For example, as the control method, a control method described in Patent Literature 1 which is a previous application of the present applicant can be used.

For example, when the force control is applied, a so-called power assist control in which the actuators of the first rotational axis portion 5311a to the sixth rotational axis portion 5311f are driven so that the supporting unit 120 smoothly moves by an external force received from the operator in a direct operation performed by the operator. As a result, it is possible to move the microscope unit 110 with relatively less force when the operator grips the microscope unit 110 to directly move the position of the microscope unit 110 in the direct operation. Accordingly, it is possible to move the microscope unit 110 with a more intuitive and simpler operation, thereby making it possible to improve convenience of the operator.

Alternatively, when the position control is applied, the operator performs an appropriate operation input through an input device (not illustrated), such that the control device 150 may appropriately control the driving of the actuators of the first rotational axis portion 5311a to the sixth rotational axis portion 5311f according to the operation input to control the position and the posture of the microscope unit 110. As the input device, it is preferable that an input device which can be operated by the operator even when the operator is holding a surgical tool, such as a foot switch or the like be applied in consideration of convenience of the operator. In addition, the operation input may be performed in a non-contact manner based on gesture detection or gaze detection using a wearable device or a camera provided in the operating room, or based on voice detection using a microphone provided in the operating room. In this case, even the operator in a clean area can operate the observation apparatus in a dirty area with a high degree of freedom. Alternatively, the supporting unit 120 may be operated in a so-called master-slave method. In this case, the supporting unit 120 can be remotely controlled by the operator through an input device provided in a place separate from the operating room.

Hereinafter, an operation of moving, by the operator, the microscope unit 110 (that is, the imaging unit) is also referred to as a moving operation. The moving operation can include a direct operation, and can also include an operation through an input device such as a foot switch or the like, or a non-contact operation through a gesture or the like.

In addition, a brake which restricts rotation of each of the first rotational axis portion 5311a to the sixth rotational axis portion 5311f is provided in each of the first rotational axis portion 5311a to the sixth rotational axis portion 5311f. Driving of the brakes is controlled by the control device 150. For example, in a case of intending to fix the position and the posture of the microscope unit 110, the control device 150 operates the brake of each rotational axis portion. As a result, the posture of the supporting unit 120, that is, the position and the posture of the microscope unit 110 can be fixed without driving the actuator, such that it is possible to reduce power consumption. When the position and the posture of the microscope unit 110 are changed, the control device 150 may release the brake of each rotational axis portion and drive the actuator according to a predetermined control method.

In the direct operation, such an operation of the brake can be switched according to an instruction input by the operator through the operation mode SW of the operating unit 5307 as described above. The brakes are simultaneously released according to the control by the control device 150 in accordance with the instruction input by the operator through the operation mode SW, such that the operation mode of the supporting unit 120 is switched to the free mode. In addition, similarly, the brakes are simultaneously driven according to the control by the control device 150 in accordance with the instruction input by the operator through the operation mode SW, such that the operation mode of the supporting unit 120 is switched to the fixed mode.

As the brake provided in each of the first rotational axis portion 5311a to the sixth rotational axis portion 5311f, various brakes used in a general observation apparatus may be applied, and a detailed mechanism thereof is not limited. For example, the brakes may be mechanically driven, or may be electromagnetic brakes which are electrically driven.

The illustrated configuration of the supporting unit 120 is only an example. The number and a shape (length) of rings constituting the supporting unit 120, the number of rotational axis portions, positions at which the rotational axis portions are disposed, directions of the rotational axes, and the like may be appropriately designed so that a desired degree of freedom can be implemented. However, as described above, in order to freely move the microscope unit 110, it is preferable to configure the supporting unit 120 to have at least six degrees of freedom. Further, as the supporting unit 120, the same configuration as that of a supporting unit of various known observation apparatuses may be applied. For example, in the supporting unit 120, an X-Y device which can move the microscope unit 110 in the X-axis direction and the Y-axis direction in the horizontal plane may be provided, instead of the second rotational axis portion 5311b and the third rotational axis portion 5311c.

### (Control Device)

The control device 150 controls an operation of the observation apparatus 10. In addition, the control device 150 also controls an operation of the display device 20. In other words, the control device 150 can collectively control an operation of the observation system 1.

The control device 150 is constituted by, for example, a processor such as a central processing unit (CPU), a digital signal processor (DSP), or the like, a control board on which the processor and a storage element such as a memory or the like are mounted together, or the like. The processor constituting the control device 150 performs a calculation processing according to a predetermined program, such that the respective functions of the control device 150 are implemented.

The control device 150 operates the actuators of the first rotational axis portion 5311a to the sixth rotational axis portion 5311f according to a predetermined control method, thereby controlling the operation of the supporting unit 120. Further, the control device 150 controls the driving of the brake provided in each rotational axis portion of the supporting unit 120 according to the operation input by the operator through the operating unit 5307 described above, thereby switching the operation mode of the supporting unit 120 described above. In addition, the control device 150 appropriately drives the optical system of the imaging unit of the microscope unit 110 according to the operation input by the operator through the operating unit 5307 described above, thereby adjusting the enlargement magnification and the focal point of the microscope unit 110.

Further, the control device 150 performs various image processings such as a gamma correction processing, a white balance adjustment processing, an enlargement processing associated with an electronic zoom function, an inter-pixel correction processing, and the like on video data transmitted from the imaging unit of the microscope unit 110. That is, the control device 150 has a function as a camera control unit (CCU). In the image processing, various image processings which are generally performed to display a video on the display device 20 may be performed. The control device 150 transmits the video data subjected to various image processings to the display device 20, and controls the display device 20 to display the video captured by the microscope unit 110. It should be noted that communication between the control device 150 and the display device 20 may be implemented in various known wired or wireless manners.

Hereinafter, the video data acquired by the imaging unit of the microscope unit 110 are also referred to as captured video data, and the video data (that is, video data after the various image processings are performed on the captured video data) displayed on the display device 20 are also referred to as displayed video data.

Hereinabove, the configuration of the observation system 1 and the observation apparatus 10 according to the first embodiment has been described with reference to FIG. 1. It should be noted that the configuration of the observation apparatus 10 according to the first embodiment is not limited to that described above. As long as the observation apparatus 10 according to the first embodiment is configured to be able to perform an observation visual field correction processing to be described later, other components of the observation apparatus 10 may be arbitrarily changed. That is, the observation apparatus 10 according to the first embodiment may have the same configuration as those of various known observation apparatuses, except that the observation visual field correction processing to be described later is performed.

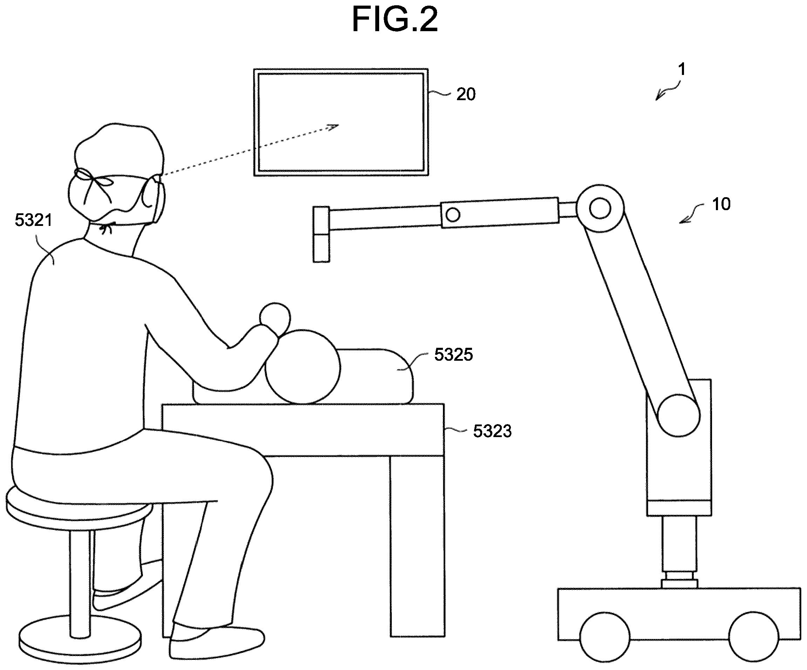

Here, FIG. 2 is a view illustrating a state in which a surgical operation using the observation system 1 illustrated in FIG. 1 is performed. FIG. 2 schematically illustrates a state in which an operator 5321 performs a surgical operation on a patient 5325 on a patient bed 5323 by using the observation system 1. It should be noted that the control device 150 is not illustrated and the observation apparatus 10 is illustrated in a simplified manner in FIG. 2 for simplification.

As illustrated in FIG. 2, at the time of performing the surgical operation, by using the observation system 1, an image of an affected part captured by the observation apparatus 10 is enlarged and displayed on the display device 20 installed on a wall surface of the operating room. The display device 20 is installed at a position at which the display device faces the operator 5321, and the operator 5321 performs various treatments on the affected portion while observing a state of the affected part through the video displayed on the display device 20.

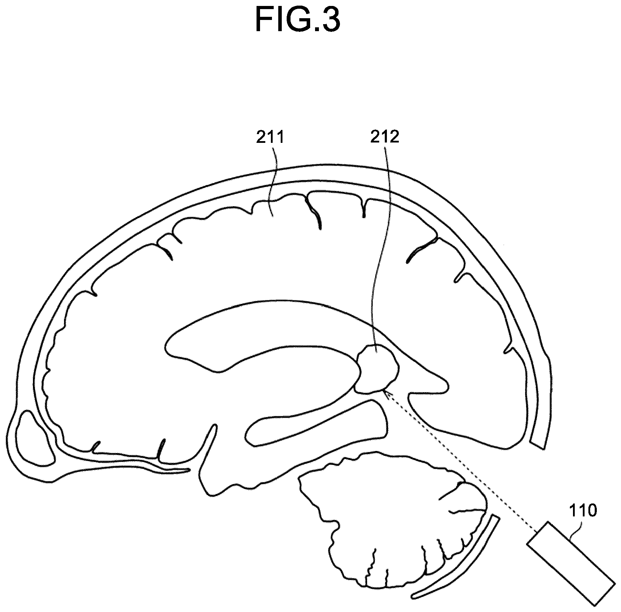

Here, the observation system 1 is used to enlarge and observe the affected part in, for example, microsurgery such as neurosurgery. In the neurosurgery, for example, there is a need to observe an affected part 212 (for example, a tumor, an aneurysm, or the like) deep in a brain 211 by using the microscope unit 110 disposed outside the brain 211 as illustrated in FIG. 3, the zoom operation of the microscope unit 110 is frequently performed. FIG. 3 is a view schematically illustrating an example of a positional relation between the brain 211 and the microscope unit 110 in the neurosurgery. FIG. 3 roughly illustrates the microscope unit 110 and an image capturing direction of the microscope unit 110 is represented by a dotted arrow.

In a state of enlarging and observing the affected part 212, an extremely narrow range is displayed on the display device 20 as an observation visual field. Accordingly, when the microscope unit 110 moves according to the moving operation or the enlargement magnification is further increased according to the zoom operation, a situation in which the affected part 212 is away from a region near the center of the observation visual field can frequently occur. In this case, there is a need to additionally perform the moving operation to finely adjust the position and the posture of the microscope unit 110 so that the affected part 212 is positioned in the region near the center of the observation visual field. However, it is difficult to perform such a fine adjustment by the direct operation in some cases. Meanwhile, it is considered that when the microscope unit 110 is moved by the actuator through the moving operation using the input device such as, for example, a foot switch or the like, the position and the posture of the microscope unit 110 can be finely adjusted more easily in comparison to the direct operation. In this case, however, a time for the operator or other medical staffs to perform the moving operation is required.

As such, in the surgical operation using the observation system 1, there is a possibility of an operation of additionally performing the moving operation so that the affected part 212 is included in the observation visual field is required, if the affected part 212 is away from the region near the center of the observation visual field when the observation visual field is changed by the moving operation or the zoom operation. It is apprehended that such an operation obstructs a smooth surgical operation.

Therefore, according to the first embodiment, in the observation system 1, when the observation visual field is changed by the moving operation or the zoom operation, a processing of automatically correcting the observation visual field so that the affected part 212 is included in the observation visual field is performed. As the observation visual field correction processing is performed, the operation of additionally performing the moving operation described above is not required, such that a load on the medical staffs such as the operator and the like is reduced, thereby enabling a smoother surgical operation.

Hereinafter, a functional configuration of the control device 150 for performing the observation visual field correction processing will be described.

### (1-2. Functional Configuration of Control Device)

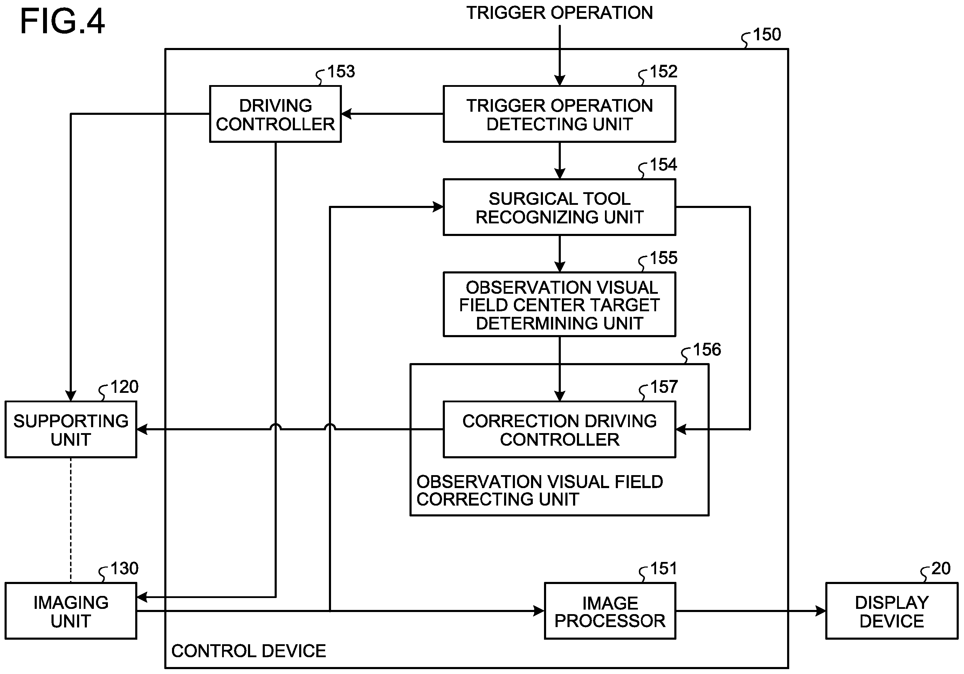

A functional configuration of the control device 150 for performing the observation visual field correction processing according to a first embodiment described above will be described in detail with reference to FIG. 4. FIG. 4 is a block diagram illustrating an example of a functional configuration of the control device 150 according to the first embodiment.

For explanation, FIG. 4 illustrates the supporting unit 120 of the observation apparatus 10, an imaging unit 130 (not illustrated in FIG. 1) included in the microscope unit 110 of the observation apparatus 10, and the display device 20 together as components other than the control device 150. In addition, FIG. 4 roughly illustrates a state in which the supporting unit 120 physically supports the imaging unit 130 by using a dotted line connecting the supporting unit 120 and the imaging unit 130 to each other.

The imaging unit 130 is mounted in the microscope unit 110 illustrated in FIG. 1. The imaging unit 130 frequently acquires captured video data obtained by capturing an image of the image capturing range during the surgical operation, and transmits the acquired video data to an image processor 151 and a surgical tool recognizing unit 154 of the control device 150 to be described later.

The display device 20 displays a video captured by the imaging unit 130 based on displayed video data generated by the image processor 151 of the control device 150 to be described later.

The control device 150 includes the image processor 151, a trigger operation detecting unit 152, a driving controller 153, the surgical tool recognizing unit 154, an observation visual field center target determining unit 155, and an observation visual field correcting unit 156 as functions thereof.

The image processor 151 performs various image processings such as a gamma correction processing, a white balance adjustment processing, an enlargement processing associated with an electronic zoom function, an inter-pixel correction processing, and the like on the captured video data transmitted from the imaging unit 130. In the image processing, various image processings which are generally performed to display a video on the display device 20 may be performed. The image processor 151 transmits the captured video data (that is, the displayed video data) subjected to various image processings to the display device 20, and displays a video based on the displayed video data (that is, the video captured by the imaging unit 130) on the display device 20.

The trigger operation detecting unit 152 detects an input of a trigger operation for performing the observation visual field correction processing. In the first embodiment, the trigger operation is an operation for changing the observation visual field, which is performed by the operator. For example, the trigger operation includes the moving operation or the zoom operation. In detail, as described above, in the observation apparatus 10, an aspect in which the operator performs the direct operation to move the microscope unit 110 in the free mode is assumed, and thus the moving operation can be an operation for switching the operation mode of the supporting unit 120 to the free mode, which is performed by the operator with respect to the operation mode SW of the operating unit 5307 illustrated in FIG. 1. Further, the zoom operation can be an operation performed by the operator with respect to the zoom SW of the operating unit 5307 illustrated in FIG. 1.

However, the first embodiment is not limited to such an example, and the trigger operation may include various operations for changing the observation visual field, which is performed by the operator. For example, when the supporting unit 120 can be operated by an operation using a foot switch, a gesture, or a voice, the trigger operation may include such an operation. Specific operations included in the trigger operation may be appropriately set by the operator, a designer of the observation system 1, or the like.

When the trigger operation is detected, the trigger operation detecting unit 152 provides, to the driving controller 153 and the surgical tool recognizing unit 154, information indicating that the trigger operation is detected and information on contents of the trigger operation.

The driving controller 153 controls the operation of the observation apparatus 10 so that the observation visual field can be changed according to the contents of the detected trigger operation. For example, when the moving operation is detected as the trigger operation, the driving controller 153 controls the operation of the actuator of each rotational axis portion of the supporting unit 120 to change a position and a posture of the imaging unit 130 according to the moving operation. Alternatively, for example, when the zoom operation is detected as the trigger operation, the driving controller 153 controls the operation of an actuator mounted on the imaging unit 130 to change the enlargement magnification by moving a position of a lens or the like constituting the optical system of the imaging unit 130 according to the zoom operation.

The surgical tool recognizing unit 154 recognizes a form of the surgical tool based on the captured video data acquired by the imaging unit 130 when the trigger operation is detected by the trigger operation detecting unit 152. Here, the form of the surgical tool includes a position and a shape of the surgical tool. For example, in the neurosurgery, a pair of retractors (brain retractors) for opening up an incision of the dura mater to expose the affected part can be used. For example, the surgical tool recognizing unit 154 recognizes a position of the pair of retractors in the captured video simultaneously with recognizing a shape of the pair of retractors based on the captured video data. Alternatively, in a case of a surgical operation performed for the purpose of resecting the affected part, an electrosurgical device for cauterizing the affected part to resect the affected part can be used. For example, the surgical tool recognizing unit 154 recognizes a position of the electrosurgical device in the captured video simultaneously with recognizing a shape of the electrosurgical device based on the captured video data.

Since the operation of the observation apparatus 10 is controlled by the driving controller 153 according to the trigger operation and the observation visual field is changed, the surgical tool recognizing unit 154 recognizes a form of the surgical tool based on the captured video data acquired by the imaging unit 130 in a state in which the observation visual field is changed.

The surgical tool recognizing unit 154 provides, to the observation visual field center target determining unit 155, information on the recognized form of the surgical tool.

The observation visual field center target determining unit 155 determines a target (observation visual field center target) to be positioned at the center of the observation visual field based on the recognized form of the surgical tool. In detail, the observation visual field center target determining unit 155 estimates a relative position of the affected part with respect to the surgical tool based on the form of the surgical tool and determines the estimated position of the affected part as the observation visual field center target.

A detailed method for estimating the position of the affected part may be appropriately set by the operator, the designer of the observation system 1, or the like according to a type, a usage, and the like of the surgical tool. For example, in general, when the retractors can be used, it is assumed that the affected part is present at a substantially intermediate position between the pair of retractors. Therefore, when the form of the pair of retractors is recognized by the surgical tool recognizing unit 154, for example, the observation visual field center target determining unit 155 estimates that the affected part is present at the substantially intermediate position between the pair of retractors and determines the estimated affected part as the observation visual field center target. Alternatively, when the affected part is resected by the electrosurgical device, usually, it is assumed that the affected part is present in a region near a distal end of the electrosurgical device. Therefore, when the form of the electrosurgical device is recognized by the surgical tool recognizing unit 154, for example, the observation visual field center target determining unit 155 estimates that the affected part is present in the region near the distal end of the electrosurgical device and determines the estimated affected part as the observation visual field center target. However, the first embodiment is not limited to such an example, and an algorithm in which the surgical tool recognizing unit 154 estimates the position of the affected part may be appropriately set according to the form of the surgical tool and a positional relation between the surgical tool and the affected part, which can be generally assumed in the case where the surgical tool is used.

The observation visual field center target determining unit 155 provides, to the observation visual field correcting unit 156, information on a relative position of the determined observation visual field center target with respect to the surgical tool.

The observation visual field correcting unit 156 corrects the observation visual field so that the observation visual field center target is positioned at substantially the center of the observation visual field based on the information on the relative position of the observation visual field center target with respect to the surgical tool, the observation visual field center target being determined by the observation visual field center target determining unit 155. In the first embodiment, the observation visual field correcting unit 156 functions as a driving controller 157 (hereinafter, referred to as a correction driving controller 157 in order to distinguish it from the driving controller 153) to control the operation of the supporting unit 120, thereby correcting the observation visual field. In detail, the correction driving controller 157 controls the operation of the supporting unit 120 to change the position and the posture of the imaging unit 130 so that the observation visual field center target determined by the observation visual field center target determining unit 155 is positioned at substantially the center of the observation visual field. At this time, since the position of the surgical tool in the captured video is recognized and the relative position of the observation visual field center target with respect to the surgical tool is identified, the correction driving controller 157 can calculate a current relative positional relation between the observation visual field center target and the imaging unit 130, and a moving amount of the imaging unit 130 for making the observation visual field center target be positioned at substantially the center of the observation visual field. Therefore, the correction driving controller 157 can correct the observation visual field by operating the supporting unit 120 so as to move the imaging unit 130 (that is, the microscope unit 110) by the calculated moving amount.

At this time, in detail, rotations around three axes (the first axis O₁, the second axis O₂, and the third axis O₃) adjacent to the distal end among the rotational axes of the supporting unit 120 can be controlled by the correction driving controller 157. This is because an angle (that is, a direction of the video) of rotation of the imaging unit 130 around a Z axis, and a position of the imaging unit 130 in the horizontal plane can be adjusted by the rotations around the rotational axes. Alternatively, when the supporting unit 120 is configured to include the X-Y device, the correction driving controller 157 may operate the X-Y device to adjust the position of the imaging unit 130 in the horizontal plane, thereby correcting the observation visual field.

As described above, according to the first embodiment, when the operator changes the observation visual field by performing an operation corresponding to the trigger operation, the position and the posture of the imaging unit 130 are controlled by the observation visual field correcting unit 156 (that is, the correction driving controller 157), and thus the observation visual field can be automatically changed so that the observation visual field center target, that is, the affected part is positioned at substantially the center of the observation visual field.

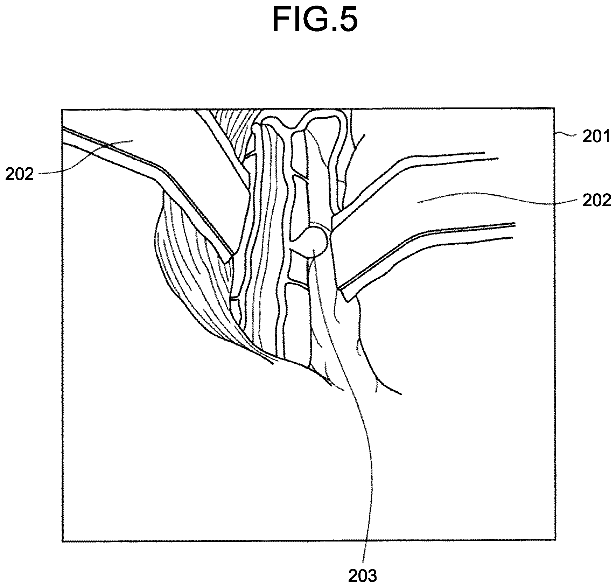

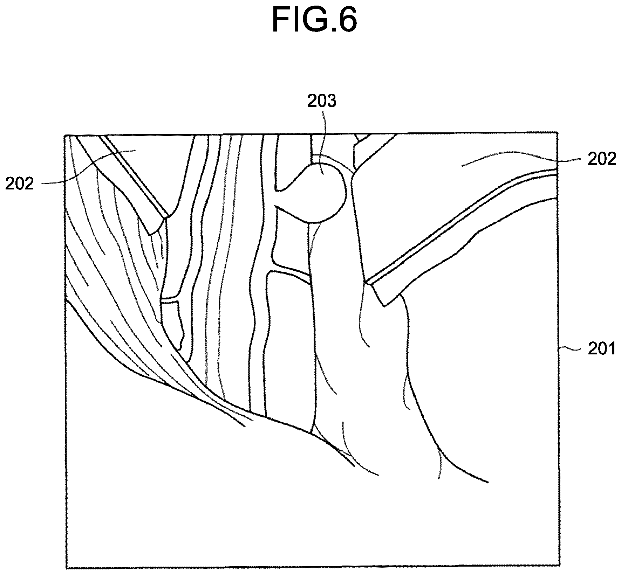

Here, an effect of the observation visual field correction processing according to the first embodiment will be described with reference to FIGS. 5 to 8. First, for comparison, an operation at the time of changing the observation visual field in the general existing observation apparatus which does not have the observation visual field correction function according to the first embodiment will be described with reference to FIGS. 5 and 6. FIGS. 5 and 6 are views for describing the operation at the time of changing the observation visual field in the general existing observation apparatus which does not have the observation visual field correction function according to the first embodiment.

FIGS. 5 and 6 schematically illustrate a displayed video (that is, the observation visual field) displayed on a display screen 201 of the display device 20. For example, first, the operator adjusts the position and the posture of the imaging unit 130 by hand, and as a result, a state in which an incision of the dura mater of the brain is opened up by a pair of retractors 202 and an aneurysm 203 which is an affected part is exposed is captured in the observation visual field as illustrated in FIG. 5. However, it is assumed that the aneurysm 203 is positioned at a position away from substantially the center of the observation visual field.

It is assumed that the operator performs the zoom operation to perform a treatment on the aneurysm 203 in this state. In this case, usually, the displayed video is enlarged around the center of the observation visual field as a base point as illustrated in FIG. 6 by the zoom operation. At this time, in the illustrated example, since the aneurysm 203 is not positioned at substantially the center of the observation visual field in the state before the enlargement illustrated in FIG. 5, the aneurysm 203 is present at a position far away from substantially the center of the observation visual field in the state after the enlargement illustrated in FIG. 6.

It is preferable for the operator that the aneurysm 203 be present at substantially the center of the observation visual field in terms of performing the treatment. Therefore, in the general existing observation apparatus, after enlargement operation is performed as illustrated in FIG. 6, the operation of finely adjusting the position and the posture of the imaging unit 130 by hand so that the aneurysm 203 is positioned at substantially the center of the observation visual field, can be required. At this time, since the enlargement magnification of the imaging unit 130 is large, the observation visual field is largely changed even with slight adjustment of the position and the posture of the imaging unit 130. Therefore, the operation of finely adjusting the position and the posture of the imaging unit 130 needs to be delicately performed. As such, in the general existing observation apparatus, there is a possibility of the delicate operation of finely adjusting the position and the posture of the imaging unit 130 at the time of the zoom operation. Therefore, it is apprehended that such an operation obstructs a smooth surgical operation.

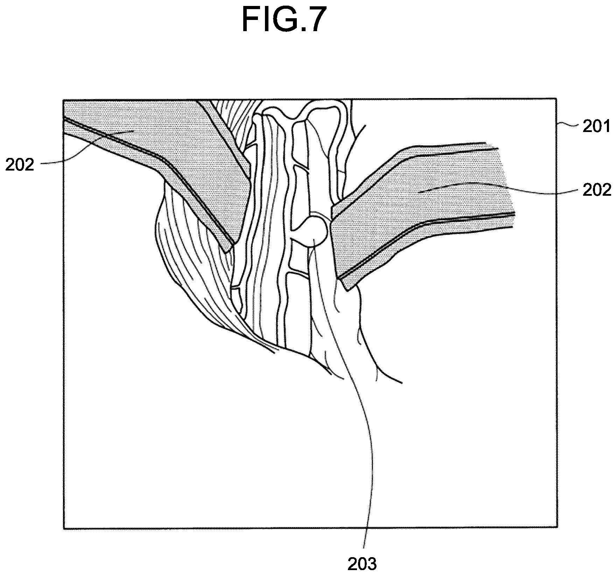

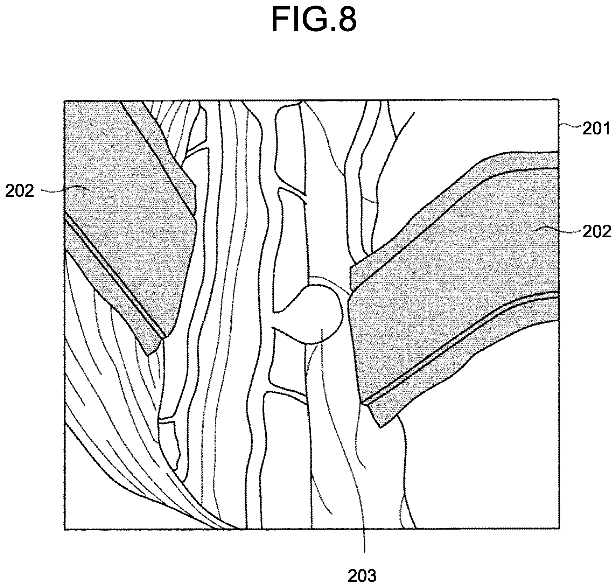

Next, an operation at the time of changing an observation visual field in the observation apparatus 10 (that is, the observation apparatus 10 having the observation visual field correction function) according to the first embodiment will be described with reference to FIGS. 7 and 8. FIGS. 7 and 8 are views for describing the operation at the time of changing the observation visual field in the observation apparatus 10 according to the first embodiment.

FIGS. 7 and 8 schematically illustrate a displayed video (that is, the observation visual field) displayed on the display screen 201 of the display device 20, similarly to FIGS. 5 and 6. For example, first, the operator adjusts the position and the posture of the imaging unit 130 by hand, and as a result, similarly to the case illustrated in FIG. 5, a state in which an incision of the dura mater of the brain is opened up by a pair of retractors 202 and an aneurysm 203 which is an affected part is exposed is captured in the observation visual field as illustrated in FIG. 7. However, it is assumed that the aneurysm 203 is positioned at a position away from substantially the center of the observation visual field.

It is assumed that the operator performs the zoom operation to perform a treatment on the aneurysm 203 in this state, similarly to the case described with reference to FIGS. 5 and 6. In this case, in the first embodiment, first, the optical system of the imaging unit 130 is operated by the driving controller 153 of the control device 150 described above to expand the observation visual field.

Then, the zoom operation is detected as the trigger operation by the trigger operation detecting unit 152. Then, the detection of the trigger operation becomes a trigger for causing the surgical tool recognizing unit 154 to recognize the form of the pair of retractors 202 (which will be expressed by application of a hatch pattern to the pair of retractors 202 in FIG. 5 for convenience). Then, the observation visual field center target determining unit 155 estimates that the aneurysm 203 which is an affected part is present at a substantially intermediate position between the pair of retractors 202 based on the recognized form of the pair of retractors 202. In addition, the observation visual field center target determining unit 155 determines the estimated position at which the aneurysm 203 is present as the observation visual field center target.

Further, the observation visual field correcting unit 156 (that is, the correction driving controller 157) controls the operation of the supporting unit 120 to adjust the position and the posture of the imaging unit 130 so that the determined observation visual field center target is positioned at substantially the center of the observation visual field (in other words, so that the aneurysm 203 is positioned at substantially the center of the observation visual field). As a result of performing a series of processings described above, the observation visual field is automatically adjusted so that the aneurysm 203 is positioned at substantially the center of the observation visual field in the observation visual field after enlargement as illustrated in FIG. 8.

As such, in the observation apparatus 10 according to the first embodiment, the observation visual field can be automatically adjusted so that the aneurysm 203 is positioned at substantially the center of the observation visual field without additionally performing the operation of finely adjusting the position and the posture of the imaging unit 130 at the time of the zoom operation. Accordingly, a smooth surgical operation can be realized.

Here, the case where the zoom operation is the trigger operation has been described as an example. However, in the first embodiment, the trigger operation may be another operation as described above. For example, the trigger operation may also be the moving operation. In this case, for example, an use aspect, in which the position and the posture of the imaging unit 130 are roughly adjusted so that the surgical tool and the affected part are included in the observation visual field according to a direct operation by the operator in a setting stage when starting the surgical operation, and then the position and the posture of the imaging unit 130 are finely adjusted so that the affected part is positioned at substantially the center of the observation visual field by the observation visual field correction processing, can be assumed.

Further, hereinabove, the retractors and the electrosurgical device have been described as an example of the surgical tool recognized by the surgical tool recognizing unit 154, but the first embodiment is not limited thereto. A target recognized by the surgical tool recognizing unit 154 may be any surgical tool. For example, the surgical tool may also be an anastomotic clip. The anastomotic clip includes a pair of holders, and is used in a manner in which the pair of holders hold portions of a blood vessel between which a cut portion of the blood vessel to be anastomosed is present at the time of anastomosis of the blood vessel. At the time of the anastomosis of the blood vessel, the portion to be anastomosed (that is, the cut portion of the blood vessel) can be enlarged and observed by using the imaging unit 130. Therefore, when the anastomotic clip is recognized as the surgical tool by the surgical tool recognizing unit 154, the observation visual field center target determining unit 155 may estimate a substantially intermediate position between the pair of holders of the anastomotic clip as the position of the affected part and determine the estimated position as the observation visual field center target. By doing so, the observation visual field can be automatically corrected so that the portion to be anastomosed is positioned at substantially the center of the observation visual field by the observation visual field correcting unit 156.

Further in the example described above, a series of processings from the surgical tool recognizing unit 154 to the observation visual field correcting unit 156 are performed after the processing of enlarging the displayed video is performed according to the zoom operation. However, the first embodiment is not limited thereto. An order in which these processings are performed may be arbitrarily set. For example, contrary to the example described above, when the trigger operation is detected, the observation visual field may be automatically changed so that the affected part is positioned at substantially the center of the observation visual field first and then the displayed video may be enlarged around substantially the center of the observation visual field as the center. Alternatively, these processings may be performed in parallel, or, for example, when the trigger operation is detected, an automatic change of the observation visual field, in which the displayed video is enlarged and simultaneously the affected part is positioned at substantially the center of the observation visual field, may be performed.

Further, in the first embodiment, the surgical tool recognition processing performed by the surgical tool recognizing unit 154 and the observation visual field center target determination processing performed by the observation visual field center target determining unit 155 may be performed at any time before the trigger operation is detected. In this case, when the trigger operation is detected, the correction of the observation visual field can be performed based on the most recently determined observation visual field center target. In this case, since there is no need to perform the surgical tool recognition processing and the observation visual field center target determination processing again after the trigger operation is detected, a time required to perform the processings can be reduced, thereby making it possible to more rapidly correct the observation visual field.

It should be noted that FIG. 4 illustrates only the functions associated with the observation visual field correction processing according to the first embodiment among functions of the control device 150, for explanation. The control device 150 can have various functions that a control device of an observation apparatus generally has, in addition to the illustrated functions. For example, the image processor 151 and the driving controller 153 can perform a processing of generating displayed video data based on captured video data and a processing of controlling operations of the supporting unit 120 and the imaging unit 130 according to the operation by the operator, respectively, not only when the observation visual field correction processing is performed (that is, not only when the trigger operation is detected), but also at a normal time.

Here, as described above, Patent Literature 2 discloses an endoscope apparatus including position detecting means which acquires position information of a surgical tool based on a video captured by an endoscope, and control means which drives an imaging optical system of the endoscope to change an image capturing range so that a distal end portion of the surgical tool is located at substantially the center of a displayed video, based on the position information. In the endoscope apparatus, the image capturing range can be automatically changed so that the distal end portion of the surgical tool is within the image capturing range of the endoscope at all times. There is a possibility that even when the technology described in Patent Literature 2 is applied to the observation apparatus 10 as it is, an appropriate observation visual field is obtained.

However, in the technology described in Patent Literature 2, an image capturing range of an endoscope is automatically changed so that a distal end portion of a surgical tool is within the image capturing range of the endoscope at all times. Therefore, when the surgical tool is moved during a treatment, an observation visual field is changed accordingly, which results in frequent movement of the observation visual field. Further, since the distal end portion of the surgical tool is a target to be included in the image capturing range, a situation in which the affected part itself is not included in the image capturing range can occur depending on a type of movement of the surgical tool. Therefore, in the technology described in Patent Literature 2, an appropriate observation visual field may not necessarily be obtained and it is apprehended that the operator is difficult to smoothly perform the treatment.

In this regard, in the first embodiment, as described above, when the trigger operation is detected, the observation visual field is corrected (that is, the observation visual field is changed). Therefore, an unnecessary movement of the observation visual field does not occur. Further, the surgical tool itself is not a target to be included in the observation visual field, and the observation visual field center target (that is, the affected part) estimated based on the form of the surgical tool is a target to be included in the observation visual field. Accordingly, the affected part can be more certainly included in the observation visual field. As such, according to the first embodiment, a more appropriate observation visual field can be obtained in comparison to an existing technology such as, for example, the technology described in Patent Literature 2. As a result, a smoother surgical operation can be realized.

### (1-3. Observation Visual Field Correction Method)

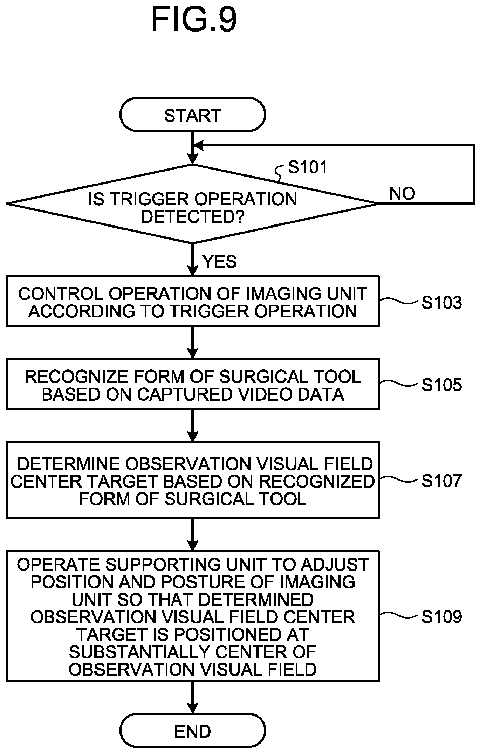

A processing procedure of an observation visual field correction method according to the first embodiment will be described with reference to FIG. 9. FIG. 9 is a flowchart illustrating an example of the processing procedure of the observation visual field correction method according to the first embodiment. It should be noted that respective processings illustrated in FIG. 9 correspond to the processings performed by the control device 150 illustrated in FIGS. 1 and 3. Details of the respective processings have already been described when describing the functional configuration of the control device 150. Therefore, a detailed description thereof will be omitted in the following description about the processing procedure of the observation visual field correction method. It should be noted that the imaging unit 130 frequently acquires the captured video when the respective processings illustrated in FIG. 9 are performed.

Referring to FIG. 9, in the observation visual field correction method according to the first embodiment, first, whether or not the trigger operation is detected is determined (Step S101). The processing in Step S101 corresponds to the processing performed by the trigger operation detecting unit 152 illustrated in FIG. 4.

When it is determined that the trigger operation is not detected in Step S101, the processing waits until the trigger operation is detected (that is, the processing in Step S101 is repeated until the trigger operation is detected) without proceeding to Step S103.

When it is determined that the trigger operation is detected in Step S101, the processing proceeds to Step S103. In Step S103, the operation of the imaging unit 130 is controlled according to the trigger operation. In Step S103, in detail, the position and the posture of the imaging unit 130 can be controlled by operating the supporting unit 120. Alternatively, the enlargement magnification can be controlled by operating a lens and the like constituting the optical system of the imaging unit 130.

Next, the form of the surgical tool is recognized based on the captured video data (Step S105). The processing in Step S105 corresponds to the processing performed by the surgical tool recognizing unit 154 illustrated in FIG. 4.

Next, the observation visual field center target is determined based on the recognized form of the surgical tool (Step S107). In Step S107, in detail, a position of the affected part is estimated based on the recognized form of the surgical tool and the estimated position of the affected part is determined as the observation visual field center target. The processing in Step S107 corresponds to the processing performed by the observation visual field center target determining unit 155 illustrated in FIG. 4.

Next, the supporting unit 120 is operated to adjust the position and the posture of the imaging unit 130 so that the determined observation visual field center target is positioned at substantially the center of the observation visual field (in other words, the observation visual field is corrected so that the determined observation visual field center target is positioned at substantially the center of the observation visual field) (Step S109). The processing in Step S109 corresponds to the processing performed by the observation visual field correcting unit 156 (that is, the correction driving controller 157) illustrated in FIG. 3.

Hereinabove, the processing procedure of the observation visual field correction method according to the first embodiment has been described. In the example illustrated in FIG. 9, the processings (the surgical tool recognition processing, the observation visual field center target determination processing, and the observation visual field correction processing) in Steps S105 to S109 are performed after the processing(the processing of controlling the operation of the imaging unit 130 according to the trigger operation) in Step S103 is performed. However, the first embodiment is not limited thereto. As described above, the processing in Step S103 and the processings in Steps S105 to S109 may be performed in an order which the reverse of the illustrated order or may be performed in parallel. In addition, the processing (the surgical tool recognition processing) in Step S105 and the processing (the observation visual field center target determining processing) in Step S107 may be performed before the processing (the trigger operation detection processing) in Step S101.

### (2. Second Embodiment)

A second embodiment of the present disclosure will be described. It should be noted that the second embodiment is different from the first embodiment described above in regard to a detailed method for correcting an observation visual field. The second embodiment is the same as the first embodiment except for the detailed method for correcting an observation visual field. Therefore, in a description of the second embodiment, matters different from the first embodiment will be mainly described and a detailed description of the same matters will be omitted.

### (2-1. Functional Configuration of Control Device)

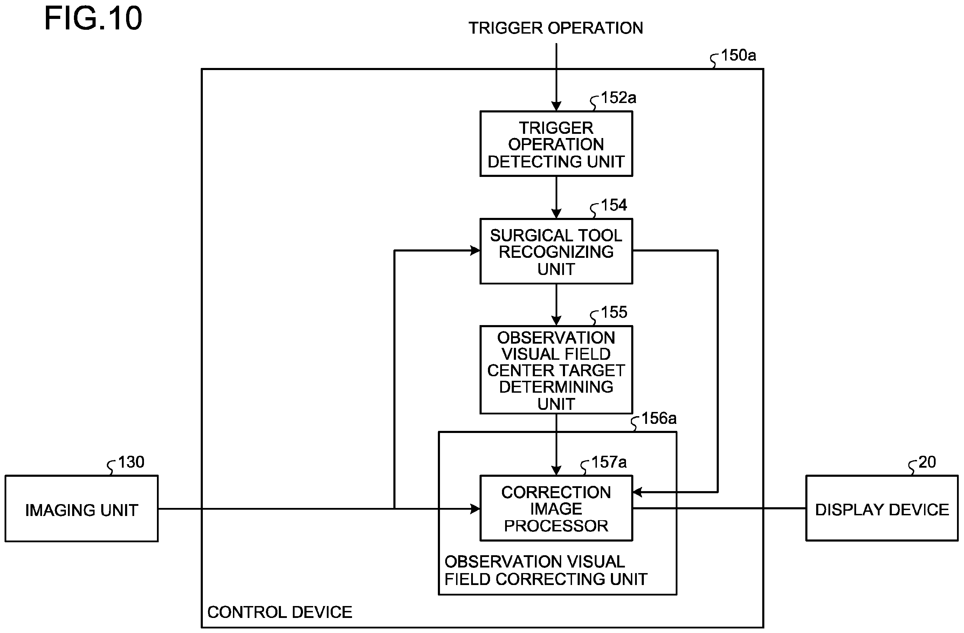

A configuration of an observation system according to the second embodiment is substantially the same as the observation system 1 according to the first embodiment described with reference to FIG. 1. However, in the second embodiment, a function of a control device 150 for performing an observation visual field correction processing is different from that of the first embodiment. Here, a functional configuration of the control device according to the second embodiment will be described with reference to FIG. 10.

FIG. 10 is a block diagram illustrating an example of a functional configuration of a control device 150a according to the second embodiment. For explanation, FIG. 10 illustrates the imaging unit 130 (not illustrated in FIG. 1) included in the microscope unit 110 of the observation apparatus 10 and the display device 20 illustrated in FIG. 1 together, as components other than the control device 150a.

In the second embodiment, the imaging unit 130 frequently acquires captured video data obtained by capturing an image of an image capturing range during a surgical operation, and transmits the acquired captured video data to a surgical tool recognizing unit 154 and an observation visual field correcting unit 156a (correcting image processor 157a) of the control device 150a to be described later. The display device 20 displays the video captured by the imaging unit 130 based on displayed video data generated by the observation visual field correcting unit 156a (correction image processor 157a) of the control device 150 to be described later.

Similarly to the control device 150, the control device 150a is constituted by, for example, a processor such as a CPU, a DSP, or the like, a control board on which the processor and a storage element such as a memory or the like are mounted together, or the like. The control device 150a includes a trigger operation detecting unit 152a, the surgical tool recognizing unit 154, an observation visual field center target determining unit 155, and the observation visual field correcting unit 156a as functions thereof. The processor constituting the control device 150a performs a calculation processing according to a predetermined program, such that the respective functions of the control device 150a are implemented. It should be noted that the functions of the surgical tool recognizing unit 154 and the observation visual field center target determining unit 155 among the functions are substantially the same as those in the first embodiment, and thus a detailed description thereof will be omitted here.

The trigger operation detecting unit 152a detects an input of a trigger operation, similarly to the first embodiment. However, in the illustrated example of the configuration, a case where the trigger operation is a zoom operation is assumed. In other words, in the illustrated example of the configuration, the trigger operation detecting unit 152a can be configured to detect only the zoom operation as the trigger operation.

The trigger operation detecting unit 152a provides, to the surgical tool recognizing unit 154, information indicating that the trigger operation is detected and information on contents of the trigger operation.

Similarly to the first embodiment, the surgical tool recognizing unit 154 recognizes a form of a surgical tool based on the captured video data. In addition, similarly to the first embodiment, the observation visual field center target determining unit 155 determines an observation visual field center target based on the form of the surgical tool.

The observation visual field correcting unit 156a corrects an observation visual field so that the observation visual field center target is positioned at substantially the center of the observation visual field based on information on a relative position of the observation visual field center target with respect to the surgical tool, the observation visual field center target being determined by the observation visual field center target determining unit 155. In the second embodiment, the observation visual field correcting unit 156a functions as the image processor 157a (hereinafter, referred to as the correction image processor 157a in order to distinguish it from the image processor 151 illustrated in FIG. 4). The correction image processor 157a generates displayed video data by performing various image processings on the captured video data, similarly to the image processor 151 in the first embodiment. However, at this time, the correction image processor 157a cuts a partial region of the captured video having the observation visual field center target as the center from the captured video by using an electronic zoom function, and enlarges the cut region at a magnification specified in the trigger operation, thereby generating the displayed video data, the observation visual field center target being determined by the observation visual field center target determining unit 155.

The correction image processor 157a transmits, to the display device 20, the displayed video data generated by performing various image processings on the captured video data, various image processings including an enlargement processing associated with the electronic zoom, and displays a video based on the displayed video data (that is, the video obtained by enlarging the captured video captured by the imaging unit 130 by the electronic zoom function) on the display device 20.

As described above, in the second embodiment, the enlargement of the observation visual field is not performed by driving an optical system of the imaging unit 130 (that is, by an optical zoom function), but is performed using the electronic zoom function by the correction image processor 157a. Further, at this time, the electronic zoom is performed around the observation visual center target as the center. As a result, the displayed video data generated by the correction image processor 157a become video data enlarged around the observation visual field center target (that is, the affected part) as the center at the magnification specified in the trigger operation.

Here, the electronic imaging observation apparatus can have the electronic zoom function even in a case of a general existing configuration. However, in a case of the electronic zoom function in the general configuration, usually, a predetermined region having substantially the center of the captured video as the center in the captured video is cut and enlarged. Therefore, similarly to the case described with reference to FIGS. 5 and 6, when the affected part is present at substantially the center of the observation visual field before the enlargement, the affected part can be present at a position far away from the center of the observation visual field after the enlargement using the electronic zoom function. Therefore, an operation of finely adjusting a position and a posture of the imaging unit 130 is required to position the affected part at substantially the center of the observation visual field. On the contrary, according to the second embodiment, since the enlargement processing is performed around the affected part as the center using the electronic zoom function as described above, similarly to the first embodiment, the operation of finely adjusting the position and the posture of the imaging unit 130 is not required, and a smooth surgical operation can be realized.

In the second embodiment, as described above, when a region corresponding to the displayed video is cut from the captured video by using the electronic zoom function, a position and a range of cutting of the region are adjusted, thereby correcting the observation visual field. When using the electronic zoom function as described above, when the enlargement magnification is large, it is apprehended that a resolution of the displayed video is degraded and it is difficult to closely observe the affected part. However, if the captured video is captured in high resolution, even when the captured video is enlarged by using the electronic zoom function, it is possible to obtain a clear displayed video. For example, even when the captured video is enlarged two times in a case of 4K, and is enlarged four times in a case of 8K by using the electronic zoom function, a full HD image quality of the displayed video can be secured. Therefore, in the second embodiment, it is preferable that the imaging unit 130 which can perform image capturing in high resolution be used in terms of securing high resolution of the displayed video.

In the second embodiment, as described above, when the region corresponding to the displayed video is cut from the captured video by using the electronic zoom function, the region having an observation visual field center target as the center can be cut, the observation visual field center target being present at a position different from substantially the center of the captured video. Therefore, there is a possibility of a displayed video reflecting a range different from that of the displayed video which is normally obtained based on the position and the posture of the imaging unit 130 during the observation visual field correction processing. Accordingly, for example, if this state is continued even after performing a moving operation to observe the affected part from a different direction thereafter, an operation for moving the imaging unit 130 performed by the operator and the displayed video that the operator is observing are out of sync in feeling, which is not preferable.

In this regard, in the second embodiment, when an explicit moving operation is input during the observation visual field correction processing (that is, when a predetermined region having the position different from substantially the center of the captured video as the center is cut and is enlarged and displayed), the observation visual field may be switched so that the observation visual field enlarged according to an aspect of the general electronic zoom function is displayed (that is, so that the predetermined region having substantially the center of the captured video as the center is cut and is enlarged and displayed). By doing so, the out of sync feeling described above can be resolved. However, it is apprehended that the operator feels confused due to the switching of the observation visual field. Therefore, in the second embodiment, an indication for notifying the operator of the fact that the observation visual field correction processing is being performed may be superimposed on the displayed video. By doing so, the operator can grasp the fact that the observation visual field correction processing is being performed, that is, the fact that the predetermined region having the position different from substantially the center of the captured video as the center is cut and is enlarged and displayed. Accordingly, the operator rarely feels confused, and it is possible to improve usability for the operator.

Further, in the example described above, the correction image processor 157a collectively performs the enlargement processing on a region having the observation visual field center target as the center by using the electronic zoom function. In other words, the correction image processor 157a simultaneously performs the observation visual field change processing around the observation visual field center target as the center, and the displayed video enlargement processing using the electronic zoom function. However, the second embodiment is not limited thereto. For example, the processings performed by the correction image processor 157a described above may be performed in stages. In detail, the correction image processor 157a may first perform the displayed video enlargement processing using the electronic zoom function, and then perform the observation visual field change processing (that is, the processing of changing the region to be cut from the captured video to the region having the observation visual field center target as the center) around the observation visual field center target as the center. In this case, the displayed video is presented to the operator in a manner in which the displayed video is enlarged first and then the observation visual field is moved, and thus the operator can more intuitively grasp the fact that the observation visual field correction processing is being performed. Alternatively, the two processings described above may be performed in a reverse order.

Further, in the second embodiment, the surgical tool recognition processing performed by the surgical tool recognizing unit 154 and the observation visual field center target determining processing performed by the observation visual field center target determining unit 155 may be performed at any time before the trigger operation is detected, similarly to the first embodiment. In this case, since there is no need to perform these processings again after the trigger operation is detected, a time required to perform the processings can be reduced, thereby making it possible to more rapidly correct the observation visual field.

Further, in the example described above, the trigger operation is limited to the zoom operation, but the second embodiment is not limited thereto. For example, even in a case of detecting the moving operation as the trigger operation, similarly to the first embodiment, it is possible to correct the observation visual field by changing a position of cutting of the captured video using the electronic zoom function as in the second embodiment. In this case, the control device 150a is configured to generate displayed video data by cutting a partial region of the captured video, for example, before the observation visual field correction processing is performed (that is, before the trigger operation is detected). At this time, before the observation visual field correction processing is performed, a predetermined region having substantially the center of the captured video as the center can be cut to generate the displayed video data. Further, when the trigger operation is detected and the observation visual field correction processing is performed, a position and a range of cutting of the captured video are changed so that a predetermined region having the observation visual field center target as the center is cut, thereby generating the displayed video data of the cut region. As described above, if the control device 150a is configured to generate the displayed video data by cutting a partial region of the captured video before the observation visual field correction processing is performed, even when an operation other than the zoom operation is set as the trigger operation, the observation visual field correction processing can be performed by appropriately changing the position and the range of the cutting of the captured video.

Further, FIG. 10 illustrates only the functions associated with the observation visual field correction processing according to the second embodiment among functions of the control device 150a, for explanation. The control device 150a can have various functions that a control device of an observation apparatus generally has, in addition to the illustrated functions. For example, the control device 150a includes an image processor 151 and a driving controller 153, similarly to the first embodiment, and the image processor 151 and the driving controller 153 can perform a process of generating displayed video data based on captured video data and a process of controlling operations of the supporting unit 120 and the imaging unit 130 according to the operation by the operator, respectively, not only when the observation visual field correction process is performed (that is, not only when the trigger operation is detected), but also in a normal time. At this time, the image processor 151 can also perform the electronic zoom. However, when the observation visual field correction processing is not performed, a predetermined region having substantially the center of the captured video as the center can be cut and enlarged to generate the displayed video data by using the electronic zoom.

### (2-2. Observation Visual Field Correction Method)

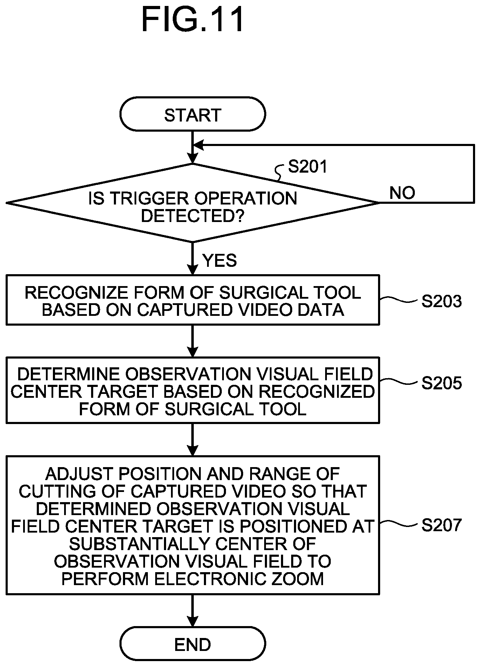

A processing procedure of the observation visual field correction method according to the second embodiment will be described with reference to FIG. 11. FIG. 11 is a flowchart illustrating an example of the processing procedure of the observation visual field correction method according to the second embodiment. It should be noted that processings illustrated in FIG. 11 correspond to the processings performed by the control device 150a illustrated in FIG. 10. Details of the processings have already been described when describing the functional configuration of the control device 150a. Therefore, a detailed description thereof will be omitted in the following description about the processing procedure of the observation visual field correction method.

Referring to FIG. 11, in the observation visual field correction method according to the second embodiment, first, whether or not the trigger operation is detected is determined (Step S201). In the second embodiment, as the trigger operation, for example, the zoom operation can be detected. The processing in Step S201 corresponds to the processing performed by the trigger operation detecting unit 152a illustrated in FIG. 10.

When it is determined that the trigger operation is not detected in Step S201, the processing waits until the trigger operation is detected (that is, the processing in Step S201 is repeated until the trigger operation is detected) without proceeding to Step S203.

When it is determined that the trigger operation is detected in Step S201, the processing proceeds to Step S203. In Step S203, the form of the surgical tool is recognized based on the captured video data (Step S203). The processing in Step S203 corresponds to the processing performed by the surgical tool recognizing unit 154 illustrated in FIG. 10.

Next, the observation visual field center target is determined based on the recognized form of the surgical tool (Step S205). In Step S205, in detail, a position of the affected part is estimated based on the recognized form of the surgical tool and the estimated position of the affected part is determined as the observation visual field center target. The processing in Step S205 corresponds to the processing performed by the observation visual field center target determining unit 155 illustrated in FIG. 10.

Next, a position and a range of cutting of the captured video are adjusted to perform the electronic zoom so that the determined observation visual field center target is positioned at substantially the center of the observation visual field (in other words, the observation visual field is corrected so that the determined observation visual field center target is positioned at substantially the center of the observation visual field) (Step S207). The processing in Step S207 corresponds to the processing performed by the observation visual field correcting unit 156a (that is, the correction image processor 157a) illustrated in FIG. 10.

Hereinabove, the processing procedure of the observation visual field correction method according to the second embodiment has been described. In the example illustrated in FIG. 11, in Step S207, the enlargement processing using the electronic zoom function is collectively performed around the observation visual field center target as the center, but the second embodiment is not limited thereto. As described above, the processing of enlarging the observation visual field using the electronic zoom function may be performed in stages. In addition, the processing (the surgical tool recognition processing) in Step S203 and the processing (the observation visual field center target determining processing) in Step S205 may be performed before the processing in Step S201.

### (3. Modified Examples)

Several modified examples of the embodiments described above will be described.

### (3-1. Another Method for Performing Surgical Tool Recognizing Processing and Observation Visual Field Center Target Determining Processing)

As described above, in the first and second embodiments, the form of the surgical tool is recognized and the position of the affected part is estimated based on the recognized form of the surgical tool. Further, the estimated position of the affected part is determined as the observation visual field center target.

However, at this time, a case where the position of the affected part is not necessarily accurately estimated can also be assumed. For example, in the example described above, it is estimated that the affected part is present at the substantially intermediate position between the pair of retractors. However, there is a possibility that the pair of retractors and the affected part do not necessarily have such a positional relation depending on a positional relation between an opening portion of an incision and the affected part, or the like.

Therefore, in the first and second embodiments, a surgical tool for indicating the position of the affected part may be set in advance, or the position indicated by the surgical tool may be determined as the observation visual field center target. For example, the surgical tool can be forceps.

When such a function is included, the surgical tool recognizing unit 154 of the control device 150 or 150a recognizes the fact that the surgical tool is the forceps and a position of a distal end of the forceps when recognizing a form of the surgical tool. Further, the observation visual field center target determining unit 155 estimates a position indicated by the distal end of the forceps as the position of the affected part, and determines the position of the affected part as the observation visual field center target.

As the function is included, even when the position of the affected part is not accurately estimated, the operator can specifically specify the position of the affected part, that is, the observation visual field center target, and thus it is possible to improve convenience of the operator. In this case, although the operator needs to additionally perform the operation of indicating the observation visual field center target in comparison to the case where the observation visual field correction processing is completely automatically performed, a load of the operation is less than the moving operation of positioning the observation visual field center target at substantially the center of the observation visual field, and the like. Therefore, even if the operation needs to be additionally performed, a smooth surgical operation is not greatly obstructed.

### (3-2. Another Example of Configuration of Observation Apparatus)

In the example of the configuration described above, the observation apparatus 10 is configured so that the actuators are mounted on all of six rotational axis portions, but the first and second embodiments are not limited thereto. For example, the observation visual field correction processing according to the first and second embodiments can be applied also to an observation apparatus configured as a balance arm. However, when the observation visual field correction processing according to the first embodiment is applied to the observation apparatus configured as the balance arm, in a supporting unit, an actuator needs to be mounted on at least a rotational axis portion which can adjust a position of a microscope unit in a horizontal plane.

Further, in the example of the configured described above, the observation apparatus 10 is an electronic imaging observation apparatus. However, the first embodiment is not limited thereto. The observation visual field correction processing according to the first embodiment can be applied also to an optical observation apparatus. For example, the optical observation apparatus can have a configuration in which an image sensor is mounted in a microscope unit, a video which is substantially the same as an image viewed by the operator with naked eyes by using the image sensor is acquired, and the acquired video can be displayed on a display device, in order for the medical staffs other than the operator share a visual field of the operator. In the optical observation apparatus having such a configuration, the observation visual field correction processing according to the first embodiment described above may be performed based on captured video data acquired to share the visual field.

### (4. Appendix)

Hereinabove, preferred embodiments of the present disclosure have been described in detail with reference to the accompanying drawings, but the technical scope of the present disclosure is not limited thereto. It will be apparent to those skilled in the art of the present disclosure that various modifications or alterations can be conceived without departing from the scope of the technical idea described in the claims and it is understood that the modifications or alterations naturally fall within the technical scope of the present disclosure.

For example, in the example illustrated in FIG. 1, the control device 150 is provided as a component different from the microscope unit 110, the supporting unit 120, and the base portion 5315, but the present disclosure is not limited thereto. For example, a processor, a control board, or the like which implements the same functions as those of the control device 150 may be disposed in the base portion 5315. In addition, a processor, a control board, or the like which implements the same functions as those of the control device 150 may be incorporated into the microscope unit 110 to integrally configure the control device 150 and the microscope unit 110. Although not illustrated in FIG. 1, an installation position of the control device 150a is also not limited.

Further, the control device 150 or 150a may not necessarily be one device, and may be implemented by a combination of a plurality of devices. In this case, the respective functions of the control device 150 or 150a are distributed to a plurality of devices having configurations which can implement the functions and the plurality of devices are cooperatively operated while transmitting and receiving various information to and from each other, such that the functions as the control device 150 or 150a can be implemented by the plurality of devices as a whole. For example, in the configuration illustrated in FIG. 1, processors, control boards, or the like may be disposed in the rotational axis portions constituting the supporting unit 120, respectively, and the plurality of processors, the plurality of control boards, or the like may cooperate with each other, thereby implementing the same functions as those of the control device 150 or 150a.

It should be noted that a computer program for implementing the respective functions of the control device 150 or 150a described above can be produced and installed in a personal computer (PC) or the like. In addition, a computer-readable recording medium in which such a computer program is stored can be provided. Examples of the recording medium include a magnetic disk, an optical disk, a magneto-optical disk, a flash memory, and the like. Further, the computer program described above may be distributed through, for example, a network, without using the recording medium.

In addition, the effects described in the present specification are merely illustrative or exemplary, and not limiting. That is, the technology according to the present disclosure may exhibit other effects apparent to those skilled in the art from the description of the present specification, in addition to or instead of the effects described above.

### Reference Signs List

- 1: OBSERVATION SYSTEM

- 10: OBSERVATION APPARATUS
- 20: DISPLAY DEVICE
- 110: MICROSCOPE UNIT
- 120: SUPPORTING UNIT (ARM PORTION)
- 130: IMAGING UNIT
- 150, 150a: CONTROL DEVICE
- 151: IMAGE PROCESSOR
- 152, 152a: TRIGGER OPERATION DETECTING UNIT
- 153: DRIVING CONTROLLER
- 154: SURGICAL TOOL RECOGNIZING UNIT
- 155: OBSERVATION VISUAL FIELD CENTER TARGET DETERMINING UNIT
- 156, 156a: OBSERVATION VISUAL FIELD CORRECTING UNIT
- 157: CORRECTION DRIVING CONTROLLER (DRIVING CONTROLLER)
- 157a: CORRECTION IMAGE PROCESSOR (IMAGE PROCESSOR)

## Claims

1. A medical observation apparatus (10) comprising:
an imaging unit (130) configured to acquire captured video data, the captured video data being data of a captured video obtained by capturing an image of an observation target;
a supporting unit (120) configured to support the imaging unit;
a trigger operation detecting unit (152) configured to detect a trigger operation, the trigger operation being a predetermined operation associated with an operation of the imaging unit;
a surgical tool recognizing unit (154) configured to recognize a form of a surgical tool included in the captured video based on the captured video data; and
an observation visual field correcting unit (156)configured to correct an observation visual field based on the form of the surgical tool recognized by the surgical tool recognizing unit when the trigger operation is detected by the trigger operation detecting unit, the observation visual field being a range of a displayed video displayed on a display device based on the captured video data;
and an observation visual field center target determining unit (156) configured to determine an observation visual field center target based on the form of the surgical tool recognized by the surgical tool recognizing unit, the observation visual field center target being a target to be positioned at the center of the observation visual field, wherein
the observation visual field correcting unit is configured to correct the observation visual field so that the observation visual field center target determined by the observation visual field center target determining unit is positioned at substantially the center of the observation visual field;
**characterised in that** the observation visual field center target determining unit is configured to estimate a relative position of an affected part with respect to a position of the surgical tool based on the form of the surgical tool and determines the position of the affected part as the observation visual field center target.

2. The medical observation apparatus according to claim 1, wherein the observation visual field correcting unit is configured to control an operation of the supporting unit and adjusts a position and a posture of the imaging unit to correct the observation visual field.

3. The medical observation apparatus according to claim 1, wherein the trigger operation is a zoom operation for enlarging the displayed video corresponding to the observation visual field, and
the observation visual field correcting unit is configured to adjust a position and a range of cutting of a predetermined region of the captured video when the predetermined region is cut and enlarged by using an electronic zoom function to correct the observation visual field.

4. The medical observation apparatus according to claim 1, wherein the surgical tool is a pair of retractors, and
the observation visual field center target determining unit is configured to estimate a substantially intermediate position between the pair of retractors as the position of the affected part.

5. The medical observation apparatus according to claim 1, wherein the surgical tool is an electrosurgical device, and
the observation visual field center target determining unit is configured to estimate a position of a distal end of the electrosurgical device as the position of the affected part.

6. The medical observation apparatus according to claim 1, wherein the surgical tool is forceps, and
the observation visual field center target determining unit is configured to estimate a position of a distal end of the forceps as the position of the affected part.

7. The medical observation apparatus according to claim 1, wherein the trigger operation is a zoom operation for enlarging the displayed video corresponding to the observation visual field, and
the observation visual field correcting unit is configured to correct the observation visual field corresponding to the displayed video enlarged according to the zoom operation.

8. The medical observation apparatus according to claim 1, wherein the trigger operation is a moving operation for moving the position and the posture of the imaging unit, and
the observation visual field correcting unit is configured to correct the observation visual field corresponding to the displayed video based on the captured video data acquired by the imaging unit after the movement according to the moving operation.

9. A computer-implemented observation visual field correction method comprising:
detecting a trigger operation, the trigger operation being a predetermined operation associated with an operation of an imaging unit (130) supported by a supporting unit (120);
recognizing a form of a surgical tool included in a captured video based on captured video data, the captured video being obtained by capturing an image of an observation target and the captured video data being data of the captured video acquired by the imaging unit; and
correcting an observation visual field based on the recognized form of the surgical tool when the trigger operation is detected, the observation visual field being a range of a displayed video displayed on a display device based on the captured video data;
determining an observation visual field center target based on the form of the surgical tool, the observation visual field center target being a target to be positioned at the center of the observation visual field, wherein
the method further comprises correcting the observation visual field so that the observation visual field center target is positioned at substantially the center of the observation visual field;
**characterised by** the method further comprising
estimating a relative position of an affected part with respect to a position of the surgical tool based on the form of the surgical tool and determining the position of the affected part as the observation visual field center target.

## Patentansprüche

1. Vorrichtung zur medizinischen Beobachtung (10), umfassend:
eine Bildgebungseinheit (130), die ausgestaltet ist, um erfasste Videodaten zu erlangen, wobei die erfassten Videodaten Daten eines erfassten Videos sind, erhalten durch Erfassen eines Bildes eines Beobachtungsziels;
eine Trägereinheit (120), die ausgestaltet ist, um die Bildgebungseinheit zu tragen;
eine Triggervorgangdetektierungseinheit (152), die ausgestaltet ist, um einen Triggervorgang zu detektieren, wobei der Triggervorgang ein vorgegebener Vorgang ist, der mit einem Vorgang der Bildgebungseinheit in Zusammenhang steht;
eine chirurgische Werkzeugerkennungseinheit (154), die ausgestaltet ist, um eine Form eines chirurgischen Werkzeugs, eingeschlossen in dem erfassten Video, basierend auf den erfassten Videodaten zu erkennen; und
eine Beobachtungssichtfeldkorrektureinheit (156), die ausgestaltet ist, um ein Beobachtungssichtfeld basierend auf der Form des chirurgische Werkzeugs, die durch die chirurgische Werkzeugerkennungseinheit erkannt wurde, zu korrigieren, wenn der Triggervorgang durch die Triggervorgangdetektierungseinheit detektiert wird, wobei das Beobachtungssichtfeld ein Bereich eines angezeigten Videos, angezeigt auf einem Anzeigegerät, basierend auf den erfassten Videodaten ist;
und eine Mittenzielbestimmungseinheit (156) des Beobachtungssichtfelds, die ausgestaltet ist, um ein Mittenziel des Beobachtungssichtfelds basierend auf der Form des chirurgische Werkzeugs zu bestimmen, die durch die chirurgische Werkzeugerkennungseinheit erkannt wurde, wobei das Mittenziel des Beobachtungssichtfelds ein Ziel ist, das in der Mitte des Beobachtungssichtfelds zu positionieren ist, wobei
die Beobachtungssichtfeldkorrektureinheit ausgestaltet ist, um das Beobachtungssichtfeld zu korrigieren, so dass das Mittenziel des Beobachtungssichtfelds, welches durch die Mittenzielbestimmungseinheit des Beobachtungssichtfelds bestimmt wird, im Wesentlichen in der Mitte des Beobachtungssichtfelds positioniert wird;
**dadurch gekennzeichnet, dass** die Mittenzielbestimmungseinheit des Beobachtungssichtfelds ausgestaltet ist, um eine relative Position eines beeinflussten Teils in Bezug auf eine Position des chirurgischen Werkzeugs basierend auf der Form des chirurgischen Werkzeugs zu schätzen, und die Position des beeinflusste Teils als Mittenziel des Beobachtungssichtfelds bestimmt.

2. Vorrichtung zur medizinischen Beobachtung nach Anspruch 1, wobei die Beobachtungssichtfeldkorrektureinheit ausgestaltet ist, um einen Vorgang der Trägereinheit zu steuern, und eine Position und eine Stellung der Bildgebungseinheiten anpasst, um das Beobachtungssichtfeld zu korrigieren.

3. Vorrichtung zur medizinischen Beobachtung nach Anspruch 1, wobei der Triggervorgang ein Zoomvorgang zur Vergrößerung des angezeigten Videos ist, das dem Beobachtungssichtfeld entspricht, und
die Beobachtungssichtfeldkorrektureinheit ausgestaltet ist, um eine Position und einen Schneidbereich einer vorgegebenen Region des erfassten Videos anzupassen, wenn die vorgegebene Region geschnitten und durch Verwendung einer elektronischen Zoomfunktion vergrößert wird, um das Beobachtungssichtfeld zu korrigieren.

4. Vorrichtung zur medizinischen Beobachtung nach Anspruch 1, wobei das chirurgische Werkzeug ein Spreizerpaar ist, und
die Mittenzielbestimmungseinheit des Beobachtungssichtfelds ausgestaltet ist, um eine wesentliche Zwischenposition zwischen dem Spreizerpaar als Position des beeinflussten Teils zu schätzen.

5. Vorrichtung zur medizinischen Beobachtung nach Anspruch 1, wobei das chirurgische Werkzeug eine elektrochirurgische Vorrichtung ist, und
die Mittenzielbestimmungseinheit des Beobachtungssichtfelds ausgestaltet ist, um eine Position eines distalen Endes der elektrochirurgischen Vorrichtung als Position des beeinflussten Teils zu schätzen.

6. Vorrichtung zur medizinischen Beobachtung nach Anspruch 1, wobei das chirurgische Werkzeug eine Zange bzw. Pinzette ist, und
die Mittenzielbestimmungseinheit des Beobachtungssichtfelds ausgestaltet ist, um eine Position eines distalen Endes der Zange bzw. Pinzette als Position des beeinflussten Teils zu schätzen.

7. Vorrichtung zur medizinischen Beobachtung nach Anspruch 1, wobei der Triggervorgang ein Zoomvorgang zur Vergrößerung des angezeigten Videos ist, das dem Beobachtungssichtfeld entspricht, und
die Beobachtungssichtfeldkorrektureinheit ausgestaltet ist, um das Beobachtungssichtfeld entsprechend dem angezeigten Video, das gemäß dem Zoomvorgang vergrößert wurde, zu korrigieren.

8. Vorrichtung zur medizinischen Beobachtung nach Anspruch 1, wobei der Triggervorgang ein Bewegungsvorgang zum Bewegen der Position und der Stellung der Bildgebungseinheit ist, und
die Beobachtungssichtfeldkorrektureinheit ausgestaltet ist, um das Beobachtungssichtfeld entsprechend dem angezeigten Video basierend auf den erfassten Videodaten, die durch die Bildgebungseinheit erlangt wurden, nach der Bewegung durch den Bewegungsvorgang zu korrigieren.

9. Computerimplementiertes Beobachtungssichtfeldkorrekturverfahren, umfassend:
Detektieren eines Triggervorgangs, wobei der Triggervorgang ein vorgegebener Vorgang ist, der mit einem Vorgang einer Bildgebungseinheit (130) zusammenhängt, die von der Trägereinheit (120) getragen wird;
Erkennen einer Form eines chirurgischen Werkzeugs, eingeschlossen in ein erfasstes Video, basierend auf erfassten Videodaten, wobei das erfasste Video durch Erfassen eines Bildes eines Beobachtungsziels erhalten wird und die erfassten Videodaten Daten des erfassten Videos sind, die durch die Bildgebungseinheit erlangt wurden; und
Korrigieren eines Beobachtungssichtfelds basierend auf der erkannten Form des chirurgischen Werkzeugs, wenn der Triggervorgang detektiert wird, wobei das Beobachtungssichtfeld ein Bereich eines angezeigten Videos, angezeigt auf einer Anzeigevorrichtung, basierend auf den erfassten Videodaten ist;
Bestimmen eines Mittenziels des Beobachtungssichtfelds basierend auf der Form des chirurgischen Werkzeugs, wobei das Mittenziel des Beobachtungssichtfelds ein in der Mitte des Beobachtungssichtfelds zu positionierendes Ziel ist, wobei das Verfahren des Weiteren Korrigieren des Beobachtungssichtfelds umfasst, so dass das Mittenziel des Beobachtungssichtfelds in im Wesentlichen der Mitte des Beobachtungssichtfelds positioniert wird;
**dadurch gekennzeichnet, dass** das Verfahren des Weiteren umfasst:
Schätzen einer relativen Position eines beeinflussten Teils in Bezug auf eine Position des chirurgischen Werkzeugs basierend auf der Form des chirurgischen Werkzeugs und Bestimmen der Position des beeinflussten Teils als Mittenziel des Beobachtungssichtfelds.

## Revendications

1. Appareil d'observation médicale (10) comprenant :
une unité d'imagerie (130) configurée pour acquérir des données vidéo capturées, les données vidéo capturées étant des données d'une vidéo capturée obtenue en capturant une image d'une cible d'observation ;
une unité de support (120) configurée pour supporter l'unité d'imagerie ;
une unité de détection d'opération de déclenchement (152) configurée pour détecter une opération de déclenchement, l'opération de déclenchement étant une opération prédéterminée associée à un fonctionnement de l'unité d'imagerie ;
une unité de reconnaissance d'outil chirurgical (154) configurée pour reconnaître une forme d'un outil chirurgical incluse dans la vidéo capturée sur la base des données vidéo capturées ; et
une unité de correction de champ visuel d'observation (156) configurée pour corriger un champ visuel d'observation sur la base de la forme de l'outil chirurgical reconnue par l'unité de reconnaissance d'outil chirurgical lorsque l'opération de déclenchement est détectée par l'unité de détection d'opération de déclenchement, le champ visuel d'observation étant une plage d'une vidéo affichée sur un dispositif d'affichage sur la base des données vidéo capturées ;
et une unité de détermination de cible de centre de champ visuel d'observation (156) configurée pour déterminer une cible de centre de champ visuel d'observation sur la base de la forme de l'outil chirurgical reconnue par l'unité de reconnaissance d'outil chirurgical, la cible de centre de champ visuel d'observation étant une cible devant être positionnée au centre du champ visuel d'observation, dans lequel
l'unité de correction de champ visuel d'observation est configurée pour corriger le champ visuel d'observation de sorte que la cible de centre de champ visuel d'observation déterminée par l'unité de détermination de cible de centre de champ visuel d'observation soit positionnée sensiblement au centre du champ visuel d'observation ;
**caractérisé en ce que** l'unité de détermination de cible de centre de champ visuel d'observation est configurée pour estimer une position relative d'une partie affectée par rapport à une position de l'outil chirurgical sur la base de la forme de l'outil chirurgical et détermine la position de la partie affectée comme étant la cible de centre de champ visuel d'observation.

2. Appareil d'observation médicale selon la revendication 1, dans lequel l'unité de correction de champ visuel d'observation est configurée pour commander un fonctionnement de l'unité de support et ajuste une position et une posture de l'unité d'imagerie pour corriger le champ visuel d'observation.

3. Appareil d'observation médicale selon la revendication 1, dans lequel l'opération de déclenchement est une opération de zoom pour agrandir la vidéo affichée correspondant au champ visuel d'observation, et
l'unité de correction de champ visuel d'observation est configurée pour ajuster une position et une plage de coupe d'une région prédéterminée de la vidéo capturée lorsque la région prédéterminée est coupée et agrandie en utilisant une fonction de zoom électronique pour corriger le champ visuel d'observation.

4. Appareil d'observation médicale selon la revendication 1, dans lequel l'outil chirurgical est une paire d'écarteurs, et
l'unité de détermination de cible de centre de champ visuel d'observation est configurée pour estimer une position sensiblement intermédiaire entre les deux écarteurs comme étant la position de la partie affectée.

5. Appareil d'observation médicale selon la revendication 1, dans lequel l'outil chirurgical est un dispositif électrochirurgical, et
l'unité de détermination de cible de centre de champ visuel d'observation est configurée pour estimer une position d'une extrémité distale du dispositif électrochirurgical comme étant la position de la partie affectée.

6. Appareil d'observation médicale selon la revendication 1, dans lequel l'outil chirurgical est un forceps, et
l'unité de détermination de cible de centre de champ visuel d'observation est configurée pour estimer une position d'une extrémité distale du forceps comme étant la position de la partie affectée.

7. Appareil d'observation médicale selon la revendication 1, dans lequel l'opération de déclenchement est une opération de zoom pour agrandir la vidéo affichée correspondant au champ visuel d'observation, et
l'unité de correction de champ visuel d'observation est configurée pour corriger le champ visuel d'observation correspondant à la vidéo affichée agrandie selon l'opération de zoom.

8. Appareil d'observation médicale selon la revendication 1, dans lequel l'opération de déclenchement est une opération de déplacement pour déplacer la position et la posture de l'unité d'imagerie, et
l'unité de correction de champ visuel d'observation est configurée pour corriger le champ visuel d'observation correspondant à la vidéo affichée sur la base des données vidéo capturées acquises par l'unité d'imagerie après le mouvement selon l'opération de déplacement.

9. Procédé de correction de champ visuel d'observation mis en œuvre par ordinateur comprenant :
la détection d'une opération de déclenchement, l'opération de déclenchement étant une opération prédéterminée associée à un fonctionnement d'une unité d'imagerie (130) supportée par une unité de support (120) ;
la reconnaissance d'une forme d'un outil chirurgical incluse dans une vidéo capturée sur la base de données vidéo capturées, la vidéo capturée étant obtenue en capturant une image d'une cible d'observation et les données vidéo capturées étant des données de la vidéo capturée acquises par l'unité d'imagerie ; et
la correction d'un champ visuel d'observation sur la base de la forme reconnue de l'outil chirurgical lorsque l'opération de déclenchement est détectée, le champ visuel d'observation étant une plage d'une vidéo affichée sur un dispositif d'affichage sur la base des données vidéo capturées ;
la détermination d'une cible de centre de champ visuel d'observation en fonction de la forme de l'outil chirurgical, la cible de centre de champ visuel d'observation étant une cible devant être positionnée au centre du champ visuel d'observation, dans lequel
le procédé comprend en outre la correction du champ visuel d'observation de sorte que la cible de centre de champ visuel d'observation soit positionnée sensiblement au centre du champ visuel d'observation ;
**caractérisé en ce que** le procédé comprend en outre
l'estimation d'une position relative d'une partie affectée par rapport à une position de l'outil chirurgical sur la base de la forme de l'outil chirurgical et la détermination de la position de la partie affectée comme étant la cible de centre de champ visuel d'observation.
